# EUROPEAN PATENT SPECIFICATION

(11) **EP 3 394 289 B1**
(45) Date of publication and mention of the grant of the patent: **26.03.2025**
(21) Application number: 16825423.3
(22) Date of filing: 22.12.2016
(51) Int. Cl.: C12Q 1/6883, C12Q 1/689

(54) **MICROBIAL MARKER IN SPONDYLOARTHRITIS DISEASES**
MIKROBIELLE MARKER BEI SPONDYLOARTHRITIS-ERKRANKUNGEN
MARQUEUR MICROBIEN DE LA SPONDYLARTHRITE

(30) Priority: 22.12.2015 EP 15201839; 23.06.2016 EP 16176033
(43) Date of publication of application: 31.10.2018
(73) Proprietor: VIB VZW, 9052 Gent (BE); Katholieke Universiteit Leuven K.U. Leuven R&D, 3000 Leuven (BE); Universiteit Gent, 9000 Gent (BE)
(72) Inventor: RAES, Jeroen, 2600 Berchem (BE); ELEWAUT, Dirk, 9070 Heusden (BE); TITO-TADEO, Raul Yhossef, 3020 Herent (BE); JOOSSENS, Marie, 9000 Gent (BE)
(86) International application number: PCT/EP2016/082355
(87) International publication number: WO 2017/109059

(56) References cited:
- WO-A1-2015/170979
- WO-A2-2015/013214
- CN-A- 104 546 939
- US-A1- 2015 225 784
- THIERRY SCHAEVERBEKE ET AL: "M?tag?nome digestif et spondylarthrite", JOINT BONE SPINE, vol. 80, no. 4, 1 July 2013 (2013-07-01), AMSTERDAM, NL, pages 327 - 331, XP055434219, ISSN: 1297-319X, DOI: 10.1016/j.jbspin.2013.02.005
- BAZSÓ ANNA ET AL: "Importance of intestinal microenvironment in development of arthritis. A systematic review", IMMUNOLOGIC RESEARCH, HUMANA PRESS, INC, US, vol. 61, no. 1, 19 November 2014 (2014-11-19), pages 172 - 176, XP035438261, ISSN: 0257-277X, [retrieved on 20141119], DOI: 10.1007/S12026-014-8593-1
- TANEJA VEENA: "Arthritis susceptibility and the gut microbiome", FEBS LETTERS, ELSEVIER, AMSTERDAM, NL, vol. 588, no. 22, 27 May 2014 (2014-05-27), pages 4244 - 4249, XP029083057, ISSN: 0014-5793, DOI: 10.1016/J.FEBSLET.2014.05.034
- TIINA DRELL ET AL: "Differences in Gut Microbiota Between Atopic and Healthy Children", CURRENT MICROBIOLOGY, vol. 71, no. 2, 14 April 2015 (2015-04-14), Boston, pages 177 - 183, XP055327058, ISSN: 0343-8651, DOI: 10.1007/s00284-015-0815-9
- CLARA ABRAHAM ET AL: "Interactions Between the Host Innate Immune System and Microbes in Inflammatory Bowel Disease", GASTROENTEROLOGY, ELSEVIER, AMSTERDAM, NL, vol. 140, no. 6, 3 February 2011 (2011-02-03), pages 1729 - 1737, XP028480606, ISSN: 0016-5085, [retrieved on 20110216], DOI: 10.1053/J.GASTRO.2011.02.012
- C. CASÉN ET AL: "Deviations in human gut microbiota: a novel diagnostic test for determining dysbiosis in patients with IBS or IBD", ALIMENTARY PHARMACOLOGY & THERAPEUTICS., vol. 42, no. 1, 14 May 2015 (2015-05-14), GB, pages 71 - 83, XP055351398, ISSN: 0269-2813, DOI: 10.1111/apt.13236

## Description

### FIELD OF THE INVENTION

The present invention relates to methods for detecting the presence inflammation in patients with spondyloarthritis (SpA) based on gut microbial markers.

### BACKGROUND

Arthritis is a condition in which one or more joints are inflamed. This can result in stiffness, soreness, and in many cases, swelling. Inflammatory and non-inflammatory arthritis are the two most common forms of the condition. Non-inflammatory arthritis (e.g. osteoarthritis) is most commonly found in the knees, hips, spine, and hands. Even though it's called non-inflammatory, non-inflammatory arthritis can still result in some inflammation of the joints. However, this inflammation mostly results from wear and tear. In particular, osteoarthritis results from the breakdown of cartilage. Cartilage is the slick tissue that covers and cushions the ends of the bones in a joint. Injuring a joint, breakdown of cartilage (e.g. in osteoarthritis patients) or everyday activities can contribute to non-inflammatory arthritis later in life. Being overweight and putting extra strain on the joints can also cause this.

Inflammatory arthritis diseases on the other hand are a group of autoimmune or auto-inflammatory diseases. Both autoimmune and auto-inflammatory disorders result from the immune system attacking the body's own tissues, leading to increased inflammation. Inflammatory arthritis diseases include rheumatoid arthritis, psoriatic arthritis, spondyloarthritis, juvenile idiopathic arthritis and systemic lupus erythematosus (lupus) among others. Inflammatory arthritis is characterized by pain, swelling, warmth and tenderness in the joints as well as morning stiffness that lasts for more than an hour. Because most inflammatory forms of arthritis are systemic (meaning they affect the entire body), other symptoms related to inflammation may occur in other parts of the body. Depending on the specific form of arthritis, these symptoms could include skin rashes, eye inflammation, hair loss, dry mouth and fever.

Spondyloarthritis (SpA), also known as spondyloarthropathy, is an inflammatory arthritis disease. SpA is a chronic inflammatory condition involving the axial skeleton and/or peripheral joints and is characterized by axial inflammation (sacroiliitis and spondylitis) and/or asymmetrical peripheral arthritis/enthesitis. Like with other inflammatory arthritis diseases, patients with SpA are often faced with extra-articular manifestations such as psoriasis, uveitis and inflammatory bowel disease (IBD). A reciprocal overlap exists between IBD and SpA: about 5-10% of SpA patients develop IBD. Conversely, up to 30% of IBD patients may develop SpA-like articular inflammation. Importantly, up to 50% of all SpA patients show intestinal inflammation on a microscopic level, without associated gastrointestinal symptoms. Two types of inflammation can be distinguished based on histomorphological characteristics: an acute type resembling infectious enterocolitis, and a chronic type, quite similar to early Crohn's disease (CD). Previous studies have demonstrated a clinical relationship between gut and joint inflammation in SpA, with remission of joint inflammation being associated with disappearance of gut inflammation and vice versa (Abraham & Medzhitov, 2011, Gastroenterology, 140:1729-1737).

The current hypothesis of IBD pathogenesis involves an inappropriate immune response to dysbalanced intestinal bacteria in a genetically susceptible host (Lees et al. 2011, Gut, 60:1739-1753). The reciprocity between bowel and joint inflammation and the overlap with IBD, suggests that intestinal microbiota might play a role in SpA pathogenesis as well. Dysbiosis with a decrease in intestinal microbial diversity has been consistently found in IBD patients (for example, Huttenhower et al. 2014, Immunity, 40: 843-854) and an altered intestinal microbiota composition has also been described in unaffected relatives of CD patients (Joossens et al. 2011, Gut, 60:631-637). A gut microbial profile distinct from unaffected controls was described for SpA patients (Costello et al. 2014, Arthritis & Rheumatology, Nov 21), yet the relation between gut microbial composition, gut histology and disease activity markers in SpA remains unknown.

The diagnosis of inflammatory arthritis diseases (e.g. spondyloarthritis) relies predominantly on clinical and radiological criteria which are often unreliable and misdiagnosis is common. Although there is a need for diagnostic markers for inflammatory arthritis diseases, in particular of markers allowing early diagnosis of said diseases, no specific marker has been established yet. Several potential diagnostic markers have been described. It has been described that HLA-B27 positive subjects are at higher risk than the general population of developing the disorder. However, 10% of healthy individuals are also HLA-B27 positive. Thus, false positive diagnosis may occur and the specificity of HLA-B27 for SpA is limited.

The treatment goals for inflammatory arthritis diseases are maintenance of physical function, control of disease activity and prevention of radiographic progression. Today, treatments consist primarily anti-inflammatory and long-lasting therapies which may not impact the underlying causes of the disease. Furthermore, effective preventative treatments for inflammatory arthritis diseases are virtually non-existent.

For spondyloarthritis, assessment of disease severity in patients was initially based on using single-item measures (e.g. pain, stiffness, erythrocyte sedimentation rate [ESR], C-reactive protein [CRP], patient/physician global assessment) and composite indices (e.g. Bath Ankylosing Spondylitis Disease Activity Index - BASDAI). However, both have limitations because they measure only part of disease activity, and are fully patient or physician oriented. The composite index Ankylosing Spondylitis Disease Activity Score (ASDAS) is a new instrument to measure disease activity in early spondyloarthritis and to establish thresholds as targets of treatment in patients with spondyloarthritis. It reflects disease activity from both the patient and the physician perspective, which are known to be inherently different. It includes the following items: back pain, duration of morning stiffness, patient global assessment of disease activity, peripheral pain or swelling, and an acute phase reactant, preferably CRP, alternatively ESR.

WO 2015/17097 discloses a method for diagnosing inflammatory arthritis by determining the intestinal microbiome and comparing with the microbiome of an healthy patient.

US2015/225784 discloses a method for determining likelihood of appendicitis in patient, which involves determining relative abundance of microorganisms in sample and comparing with reference value. The microorganism profile include Dialister spp.

TANEJA VEENANA et al. (2014) FEBS LETTERS, vol. 588, no. 22, pages 4244-4249 discloses the link between gut microbe profile and rheumatoid arthritis (inflammatory arthritis) and its use as biomarker for assessing risk in developing the said disease.

There is a need for improved means and methods for the treatment and/or prevention of spondyloarthritis and other inflammatory arthritis diseases. A need also exists for improved means and methods to diagnose and assess severity of these diseases and identify individuals predisposed to developing them.

### SUMMARY OF THE INVENTION

The present invention is defined by the appended claims.

In the current application, methods are provided for detecting the presence of gut inflammation associated with spondyloarthritis. This is based on the finding of a significant difference in intestinal microbial composition in SpA patients with and without microscopic gut inflammation was surprisingly found. A similar significant difference in intestinal microbial composition was found between inflamed and healthy samples. Moreover, we identified *Dialister* as a microbial marker of disease activity and as a potential important microbial species in SpA pathogenesis.

In particular, we noticed increased bacterial diversity in inflamed versus non-inflamed colonic and ileal biopsies of SpA patients. The same increased bacterial diversity was seen in inflamed biopsies of SpA patients and biopsies of healthy subjects. Overall bacterial community composition was also significantly different between inflamed and non-inflamed or healthy biopsies. This was unexpected, since the art suggested that intestinal inflammation, such as that observed in patients with IBD, is associated with lower microbial diversity. In addition, the present study is the first to identify a particular bacterial species and correlate its presence/abundance to disease activity in SpA: we found the genus *Dialister* as a microbial marker that correlates with ASDAS in both ileum and colon biopsies. Moreover, these results could be confirmed using non-invasive samples such as stool samples and oral samples.

This application provides methods to detect the presence of gut inflammation associated with SpA in a patient. The invention also provides methods to detect the presence gut inflammation in a patient with an SpA disease. The methods comprise the steps of (i) determining a gut microbiome profile for said patient and comparing said gut microbiome profile of said patient to one or more gut microbiome reference profiles, wherein said one or more gut microbiome reference profiles comprise at least one of a positive gut microbiome reference profile based on results from control subjects with gut inflammation in case the method is provided to detect gut inflammation in patients with SpA disease and a negative gut microbiome reference profile based on results from control subjects without without gut inflammation if said gut microbiome profile for said patient statistically significantly matches said positive gut microbiome reference profile for gut inflammation, then concluding that said patient has gut inflammation; and/or (iii) if said gut microbiome profile for said patient statistically significantly matches said negative gut microbiome reference profile for gut inflammation, then concluding that said patient does not have gut inflammation.

In some embodiments, a statistically significant match has a P value of 0.05 or less. In some embodiments, the gut microbiome profile is determined in a biological sample selected from a mucosal biopsy sample, a stool sample, a sample of the lumen content, an oral sample, a blood sample, a serum sample or a urine sample. In other embodiments, the gut microbiome profile may include one or more of bacterial taxa identified in said biological sample; bacterial metabolic products in said biological sample; and proteins in said biological sample. In yet other embodiments, the gut microbiome profile is based on an analysis of amplification products of DNA and/or RNA of the gut microbiota, e.g. based on an analysis of amplification products of genes coding for one or more of small subunit rRNA, etc. In some embodiments, the gut microbiome profile of said patient includes an indication of the presence and/or abundance of at least one of *Dialister* spp. In other embodiments, when the gut microbiome profile of said patient indicates the presence of *Dialister* spp., then said concluding step results in a conclusion that said SpA patient has gut inflammation

In a more particular embodiment, methods are provided to detect the presence of gut inflammation in a patient with SpA. The methods comprise the steps of (i) determining the abundance of a *Dialister* spp. in a biological sample of said patient, (ii) if said abundance for said patient is at least 10%, at least 20%, at least 30%, at least 40%, at least 50%, at least 60%, at least 70%, at least 80%, at least 90%, at least 100% higher or at least 2-fold, at least 3-fold, at least 4-fold, at least 5-fold higher or between 2-fold and 10-fold higher or between 3-fold and 7-fold higher than the abundance of said *Dialister* spp. in said biological sample of a control subject without gut inflammation, then said concluding step results in a conclusion that said patient has gut inflammation

The application also provides methods of monitoring the efficacy of a treatment for a gut inflammation in a SpA in a patient, said method comprises the steps of (i) determining the abundance of a *Dialister* spp. in a biological sample of said patient at two or more successive time points with samples collected from said patient at two or more successive time periods during said treatment, (ii) if the abundance of said *Dialister* spp. for said patient at a later time point of said two or more successive time points is at least 10%, at least 20%, at least 30%, at least 40%, at least 50%, at least 60%, at least 70%, at least 80%, at least 90%, at least 100% lower than the abundance of said *Dialister* spp. at an earlier time point of said two or more successive time points, then concluding that said treatment is efficacious.

In alternative but equivalent wordings, the present invention also relates to the use of a gut microbiome profile to assess the presence of gut inflammation in a patient SpA

Further embodiments will be found in the detailed description.

### BRIEF DESCRIPTION OF THE DRAWINGS

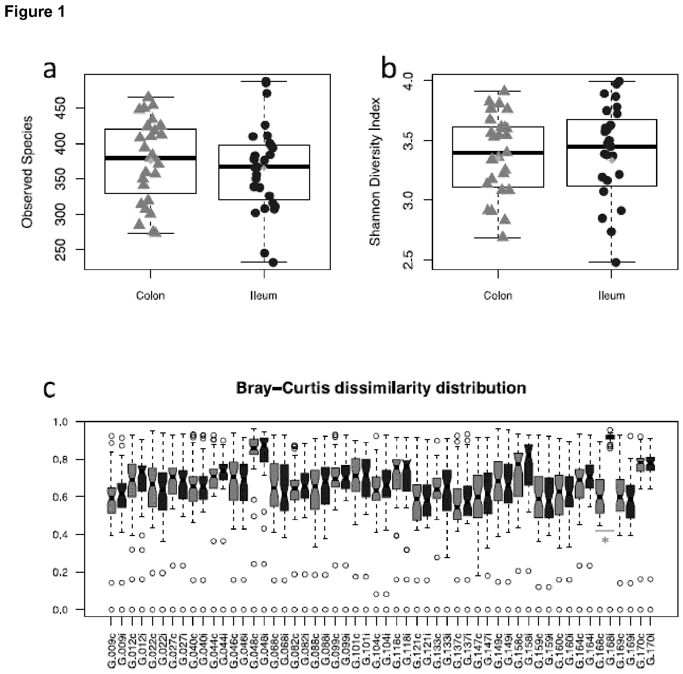

**Figure 1****. Alpha and beta diversity of the biopsies.** Microbial diversity comprises richness (observed species) and evenness (how relative abundance or biomass is distributed among species). While richness is a direct measurement of OTUs (a), the Shannon Diversity Index estimates evenness (b). Our analysis did not detect significant differences between richness and evenness of colonic and ileal biopsies. Orange diamonds are the mean in plots (a) and (b). Bray Curtis dissimilarity distances (BCD) of the colonic and ileal biopsies shows the variation in the community composition (beta diversity). In (c), we show the BCD of all pair wise comparison between a given biopsy (ileum or colon) and the rest of the biopsies. Empty circles represent outlier on the box plots, for all the samples there are outliers at 0 (Bray-Curtis distance against the same sample). After performing Welch t-test with 1000 permutations to test if the mean of the BCD of ileum and colonic biopsies of the same individual is significant different, only one comparison was significant (sample G.168 -in orange *).

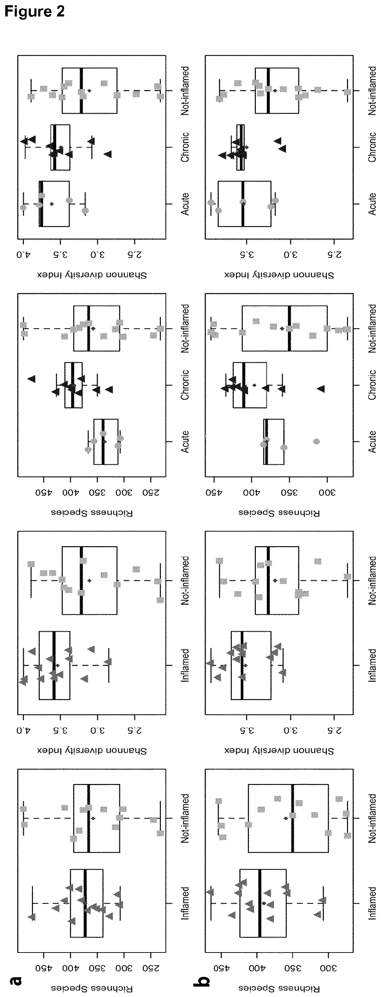

**Figure 2****. Microbial diversity and inflammation in ileal (a) and colonic (b) biopsies.** Inflamed biopsies present significant higher Shannon Diversity Index (evenness) compared to non-inflamed samples (p=0.030 for ileum and p=0.025 for colon). The Shannon Diversity Index in ileal biopsies also showed a correlation between the type of inflammation and diversity: higher in acute, followed by chronic inflammation and lower levels of diversity in non-inflamed biopsies. Microbial richness of ileal and colonic biopsies, grouped within inflammation categories, revealed higher richness in chronic than in acute and non-inflamed biopsies.

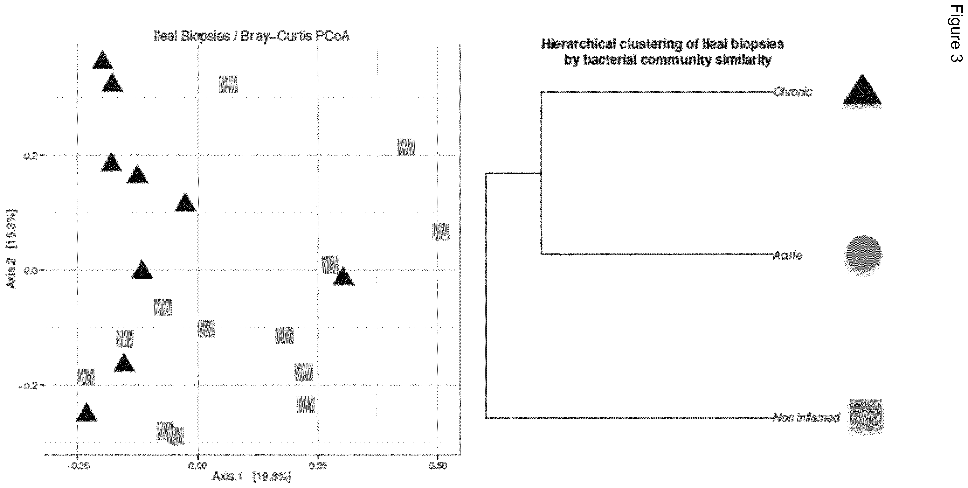

**Figure 3****. Ileal bacterial community diversity and local inflammation.** The Bray-Curtis dissimilarity distances PCoA plot of bacteria community composition does not show a clear separation or clustering by inflammation status. However, after conducting PERMANOVA, a p-value of 0.016 indicates significant differences among bacterial community composition that, through hierarchical clustering, are found to be between inflamed and non-inflamed samples.

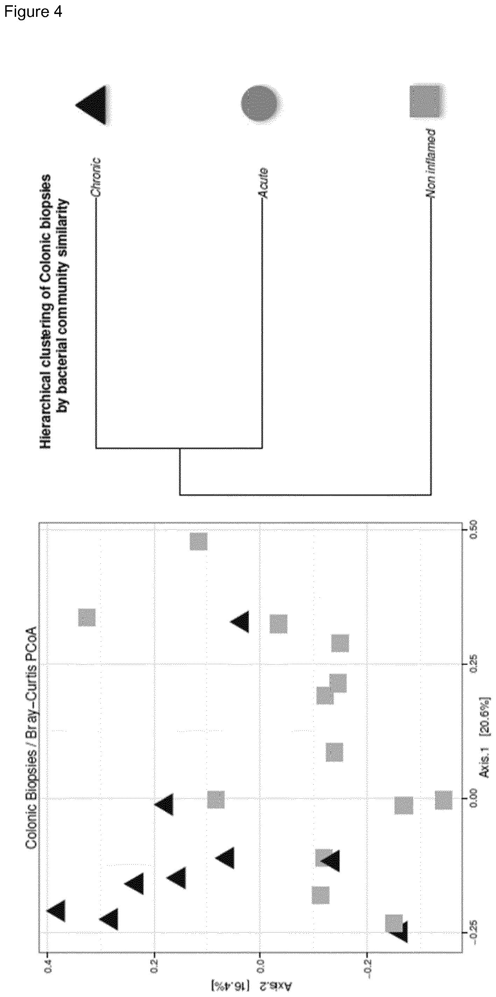

**Figure 4****. Colonic bacterial community diversity and local inflammation.** The Bray-Curtis dissimilarity distances PCoA plot of bacteria community composition does not show a clear separation or clustering by inflammation status. However, after conducting PERMANOVA, a p-value of 0.011 indicates significant differences among bacteria community composition between at least one of the inflammation status categories from the others.

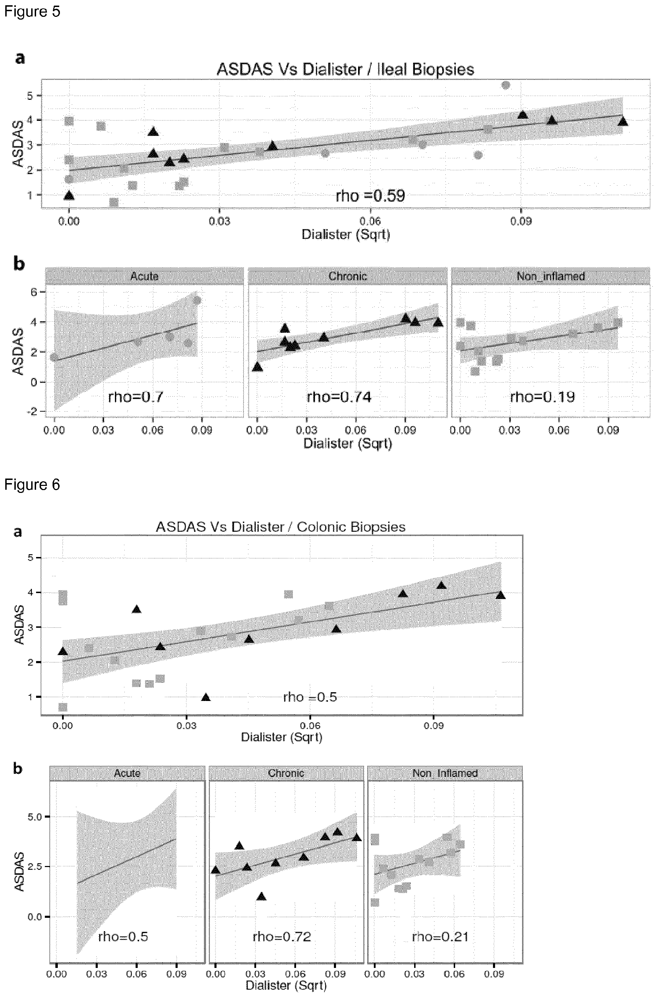

**Figure 5****. Spearman's rank correlation of the abundance of the genus *Dialister* with disease activity.** a. *Dialister* positively correlated with ASDAS values (rho = 0.59, p= 5.53E-04, q=0.026); **b.** Results divided by bowel histology: acute (rho = 0.70, p=0.350, q=1.0), chronic (rho = 0.74, p=0.035, q=0.105) and non-inflamed (rho = 0.19, p=0.802, q=1.0). *Dialister* abundance is presented by square root (Sqrt) to facilitate visualization.
**Figure 6****. Spearman's rank correlation of the abundance of the genus *Dialister* with disease activity.** a. *Dialister* positively correlates with ASDAS values (rho = 0.5, p= 0.004, q=0.167). b. Results divided by bowel histology: acute (rho = 0.5, p=0.225, q=0.675), chronic (rho = 0.72, p=0.018, q=0.054) and non-inflamed (rho = 0.21, p=0.246, q=0.63).

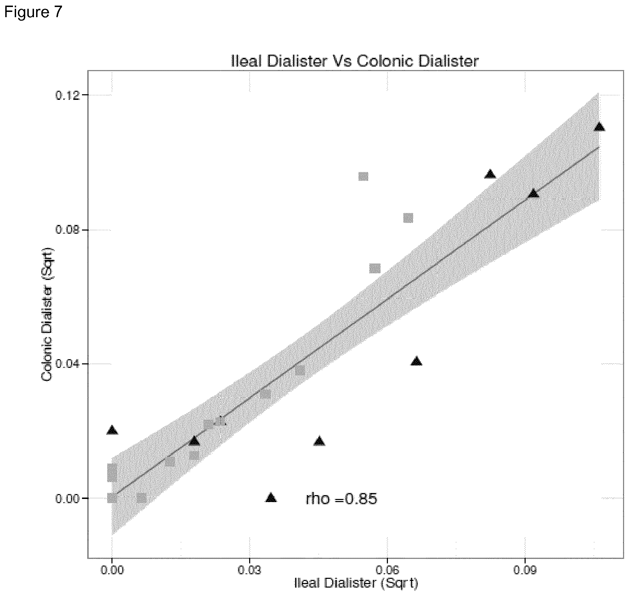

**Figure 7****. High concordance in the abundance of genus *Dialister* in ileal and colonic biopsies.** Spearman rank correlation of abundance of *Dialister* in Ileal vs colonic biopsies. (rho = 0.85 and p = 4.063e-09, q= 7.45E-06).

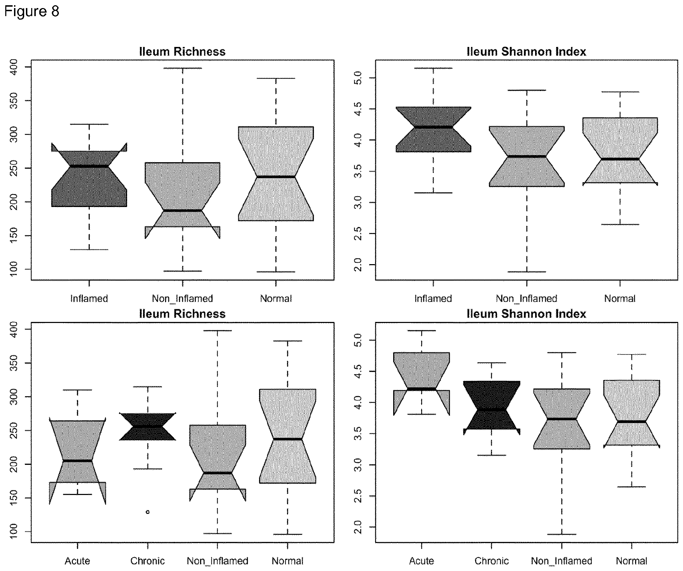

**Figure 8****. Microbial diversity and inflammation in ileal biopsies from SpA patients (Inflamed (Acute/Chronic) -Non_Inflamed) and healthy controls (Normal).** Microbial richness grouped within inflammation subtypes, revealed higher richness in chronic than in acute and non-inflamed biopsies. The Shannon Diversity Index showed a correlation between the type of inflammation and diversity: higher in acute, followed by chronic inflammation and lower levels of diversity in non-inflamed and healthy.

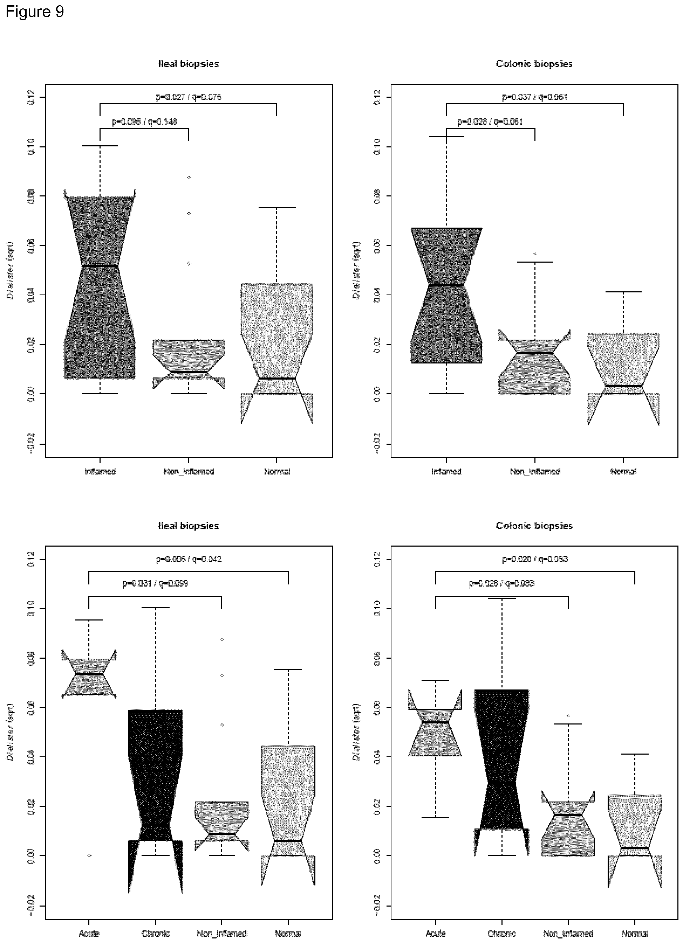

**Figure 9****. *Dialister* abundance in ileal and colonic biopsies from SpA patients and healthy controls (Normal).** T-test analysis reveals that abundance of Dialister is significantly higher in ileal and colonic biopsies from the inflamed versus non-inflamed and healthy control groups. All these comparisons remain significant after multiple test correction for a FDR <0.10. Dialister abundance is presented by square root (Sqrt) to facilitate visualization.

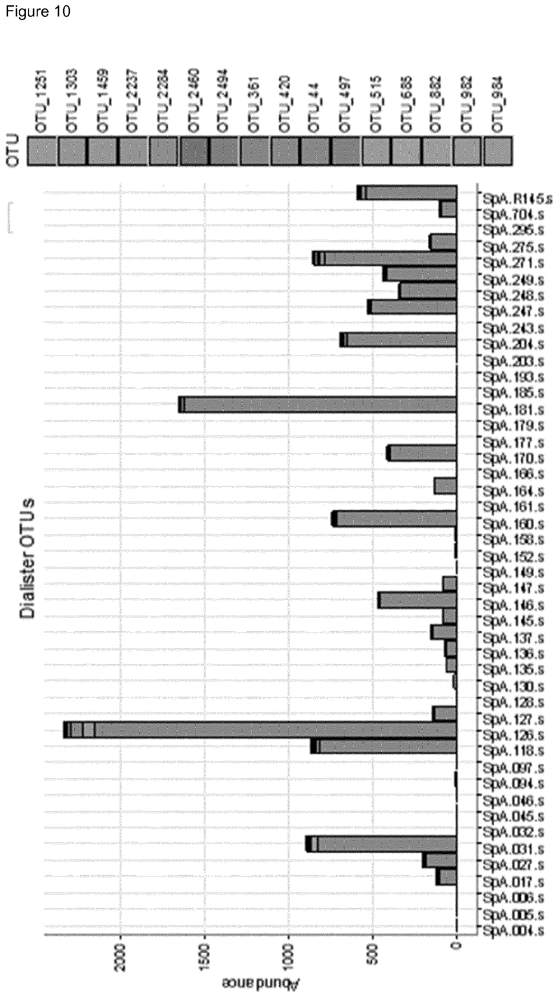

**Figure 10****. *Dialister* abundance in stool samples from SpA patients.** *Dialister* is present in 34 out of 46 stool samples from SpA patients whereby OTU_44 is the main contributor.

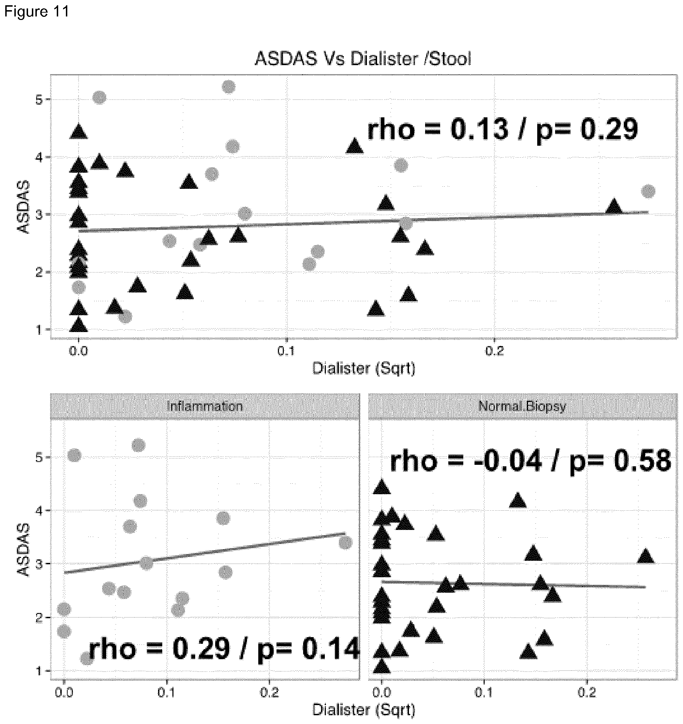

**Figure 11****. Positive *Dialister* correlation from stool samples with ASDAS.** The positive correlation value of 0.13 for all stool samples (upper panel) can be divided in 0.29 for patients with inflammation (lower left) and -0.04 for patients without inflammation (lower right).

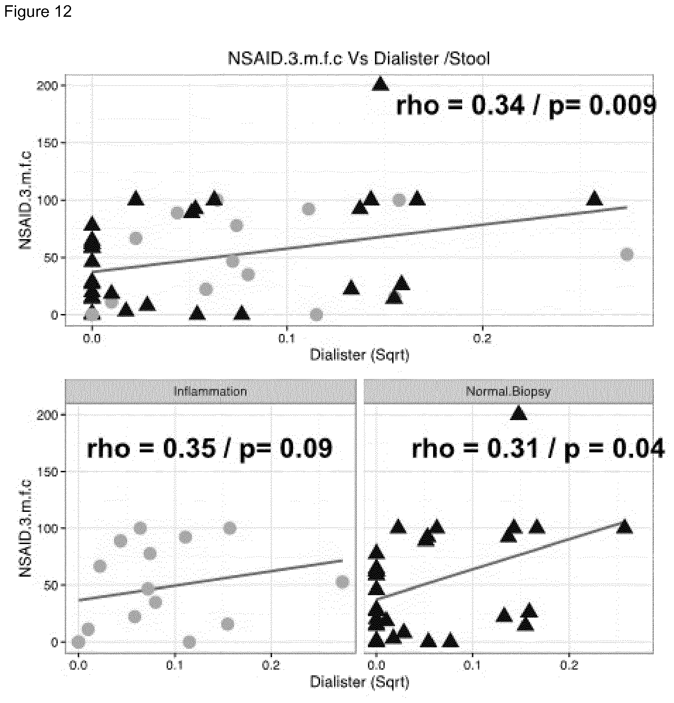

**Figure 12****. *Dialister* abundance positively correlates with levels of intake of non-steroidal anti-inflammatory drugs (NSAID).**

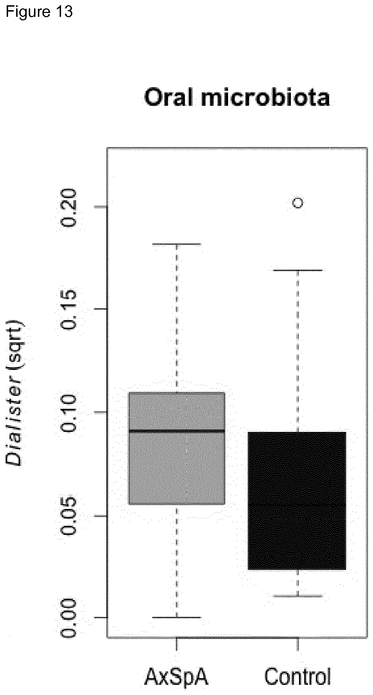

**Figure 13****. Correlation between *Dialister* abundance in the sub gingival plaque of patients with axial spondyloarthritis (AxSpA) or withouth AxSpA (control).** A reanalysis of the data of Bisanz et al 2016 revealed that the AxSpA group exhibits a higher mean abundance of *Dialister* (y-axis) in oral samples.

### DETAILED DESCRIPTION OF THE INVENTION

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated. Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Unless otherwise defined herein, scientific and technical terms and phrases used in connection with the present invention shall have the meanings that are commonly understood by those of ordinary skill in the art. Generally, nomenclatures used in connection with, and techniques of molecular and cellular biology, structural biology, biophysics, pharmacology, genetics and protein and nucleic acid chemistry described herein are those well-known and commonly used in the art. The methods and techniques of the present invention are generally performed according to conventional methods well known in the art and as described in various general and more specific references that are cited and discussed throughout the present specification unless otherwise indicated. See, for example, Sambrook et al. Molecular Cloning: A Laboratory Manual, 3th ed., Cold Spring Harbor Laboratory Press, Cold Spring Harbor, N.Y. (2001); Ausubel et al., Current Protocols in Molecular Biology, Greene Publishing Associates (1992, and Supplements to 2002); Rupp, Biomolecular crystallography: principles, Practice and Applications to Structural Biology, 1st edition, Garland Science, Taylor & Francis Group, LLC, an informa Business, N.Y. (2009); Limbird, Cell Surface Receptors, 3d ed., Springer (2004); Flow Cytometry Protocols, 2nd ed. Humana Press (2004); Antibody engineering, 2nd ed. Springer (2010).

In this application a systems biology approach was used to identify novel correlations between SpA diseases and gut microflora. For example, the studies disclosed herein successfully demonstrated a link between the composition of the gut microbiome in SpA patients with and without gut inflammation. This has led to the development of methods of assessing the propensity (risk, likelihood, etc.) SpA patients to develop gut inflammation, and methods of monitoring the progress of treatment. Furthermore, the studies disclosed herein identified *Dialister* as a microbial marker of disease activity and as a potential important microbial species in SpA pathogenesis.

As used herein, the term "inflammatory arthritis" or "inflammatory arthritis disease" refers to a group of auto-inflammatory or autoimmune diseases characterized by inflammation of the joints and often other tissues and that includes rheumatoid arthritis, juvenile rheumatoid arthritis, psoriatic arthropathy, spondyloarthritis, inflammatory bowel disease (IBD) related arthritis, juvenile idiopathic arthritis, crystal-induced arthropathies among others. It differs from non-inflammatory arthritis such as osteoarthritis in its origin. While inflammatory arthritis originates from an autoimmune and/or auto-inflammatory response during which the tissue that lines and cushions the joints is attacked by the immune system, non-inflammatory arthritis is a degenerative joint disease whereby the joint's cartilages is broken down by wear and tear.

As used herein, "a patient with inflammatory arthritis" is equivalent to the wording "a patient with an inflammatory arthritis disease", "a patient suffering from inflammatory arthritis", "a patient suffering from an inflammatory arthritis disease" or to the wording "an inflammatory arthritis patient".

As used herein, the term "spondyloarthritis (SpA)" refers to a group of closely related, but clinically heterogeneous, inflammatory arthritis diseases with common features, including inflammation of the spine, eyes, skin and gastrointestinal tract. This group is also sometimes referred to as spondylitis and spondyloarthropathies and includes ankylosing spondylitis, non-radiographic axial SpA, juvenile onset SpA, reactive arthritis (Reiter's syndrome), psoriatic arthritis, enteropathic arthritis (arthritis associated with inflammatory bowel disease or inflammatory bowel disease (IBD) related arthritis), undifferentiated spondyloarthritis, and juvenile idiopathic arthritis. Characteristics of these diseases include inflammatory arthritis of the spine, peripheral arthritis that differs from rheumatoid arthritis, extra articular manifestations of inflammatory bowel disease, arthritis and uveitis, seronegativity for rheumatoid factor and some degree of heritability, including the presence of the gene HLA-B27.

As used herein, "a patient with spondyloarthritis" is equivalent to the wording "a patient suffering from spondyloarthritis" or to the wording "a SpA patient". SpA is the abbreviation of spondyloarthritis.

As used herein, the term "gut" generally comprises the stomach, the colon, the small intestine, the large intestine, cecum and the rectum. In addition, regions of the gut may be subdivided, e.g. the right versus the left side of the colon may have different microflora populations due to the time required for digesting material to move through the colon, and changes in its composition in time. Synonyms include bowel, the gastrointestinal tract, or possibly the digestive system, although the latter is generally also understood to comprise the mouth, esophagus, etc.

The wording "gut inflammation" is equivalent to the wording "microscopic gut inflammation" as used herein and refers to an inflammatory response in the gut as defined above. The inflammation can affect the entire gastrointestinal tract, can be more limited to for example the small intestine or large intestine but can also be limited to specific components or structures such as the bowel walls.

The term "inflammation" refers to complex but to the skilled person well known biological response of body tissues to harmful stimuli, such as pathogens, damaged cells, or irritants. However, inflammation is not a synonym for infection. Infection describes the interaction between the action of microbial invasion and the reaction of the body's inflammatory response - the two components are considered together when discussing an infection, and the word is used to imply a microbial invasive cause for the observed inflammatory reaction. Inflammation on the other hand describes purely the body's immunovascular response, whatever the cause may be. Inflammation is a protective response involving immune cells, blood vessels, and molecular mediators. The function of inflammation is to eliminate the initial cause of cell injury, clear out necrotic cells and tissues damaged from the original insult and the inflammatory process, and to initiate tissue repair. The classical signs of inflammation are heat, pain, redness, swelling, and loss of function. Inflammation is a generic response, and therefore it is considered as a mechanism of innate immunity, as compared to adaptive immunity, which is specific for each pathogen. Inflammation can be classified as either acute or chronic. Acute inflammation is the initial response of the body to harmful stimuli and is achieved by the increased movement of plasma and leukocytes (especially granulocytes) from the blood into the injured tissues. A series of biochemical events propagates and matures the inflammatory response, involving the local vascular system, the immune system, and various cells within the injured tissue. Prolonged inflammation, known as chronic inflammation, leads to a progressive shift in the type of cells present at the site of inflammation, such as mononuclear cells, and is characterized by simultaneous destruction and healing of the tissue from the inflammatory process.

The term "joint inflammation" refers to the presence of inflammation or of an inflammatory response in the joints. It involves the body's immune systems attacking the joints. The inflammation can involve the whole joint, including the bone and the cartilage, but can also be more specific to particular components such as the synovial membrane, the synovium, the joint capsule or the connecting muscles. It typically results in warm, swollen, and painful joints.

As used herein, the term "joint" or "articulation" (or "articular surface") is the connection made between bones in the body which link the skeletal system into a functional whole. They are constructed to allow for different degrees and types of movement. Non-limiting examples of joints are knee joints, elbow joints, joints of the shoulder, joints of the hand, wrist joints, axillary articulations, sternoclavicular joints, vertebral articulations, temporomandibular joints, sacroiliac joints, hip joints, articulations of foot.

As used herein, the term "microflora" refers to the collective bacteria in an ecosystem of a host (e.g. an animal, such as a human) or in a single part of the host's body, e.g. the gut. An equivalent term is "microbiota".

As used herein, the term "microbiome" refers to the totality of bacteria, their genetic elements (genomes) in a defined environment, e.g. within the gut of a host, the latter then being referred to as the "gut microbiome".

As used herein, the term "patient" or "individual" or "subject" typically denotes humans, but may also encompass reference to non-human animals, preferably warm-blooded animals, more preferably mammals, such as, e.g. non-human primates, rodents, canines, felines, equines, ovines, porcines, and the like. A "patient" for the purpose of this application, is in need of treatment for an inflammatory arthritis disease, particularly spondyloarthritis, for joint and/or gut inflammation, more particularly for joint and/or gut inflammation linked to inflammatory arthritis, even more particularly for joint and/or gut inflammation linked to spondyloarthritis.

Also, the term "gut microbiome profile" is equivalent in wording as "gut microbiome signature" and these wordings are used interchangeably herein. A gut microbiome profile represent the presence, absence or the abundance of one or more of bacterial taxa identified in a biological sample; bacterial metabolic products in said biological sample; and proteins in said biological sample. In the context of the present application, the said bacterial metabolic products and said proteins are derived from *Dialister* spp. and/or *Dialister* spp. are one of the said bacterial taxa identified in said biological sample. The gut microbiome profile can be determined based on an analysis of amplification products of DNA and/or RNA of the gut microbiota, e.g. based on an analysis of amplification products of genes coding for one or more of small subunit rRNA, etc. and/or based on an analysis of proteins and/or metabolic products present in the biological sample. Gut microbiome profiles may be "compared" by any of a variety of statistical analytic procedures.

"Operational taxonomic unit" ("OTU", plural OTUs)" refers to a terminal leaf in a phylogenetic tree and is defined by a specific genetic sequence and all sequences that share sequence identity to this sequence at the level of species. A "type" or a plurality of "types" of bacteria includes an OTU or a plurality of different OTUs, and also encompasses a strain, species, genus, family or order of bacteria. The specific genetic sequence may be the 16S rRNA sequence or a portion of the 16S rRNA sequence or it may be a functionally conserved housekeeping gene found broadly across the eubacterial kingdom. OTUs share at least 95%, 96%, 97%, 98%, or 99% sequence identity. OTUs are frequently defined by comparing sequences between organisms. Sequences with less than 95% sequence identity are not considered to form part of the same OTU.

The term "sequence identity" as used herein refers to the extent that sequences are identical on a nucleotide-by-nucleotide basis over a window of comparison. Thus, a "percentage of sequence identity" is calculated by comparing two optimally aligned sequences over the window of comparison, determining the number of positions at which the identical nucleic acid base (e.g., A, T, C, G, I) occurs in both sequences to yield the number of matched positions, dividing the number of matched positions by the total number of positions in the window of comparison (i.e. the window size), and multiplying the result by 100 to yield the percentage of sequence identity. A gap, i.e. a position in an alignment where a residue is present in one sequence but not in the other is regarded as a position with non-identical residues. Determining the percentage of sequence identity can be done manually, or by making use of computer programs that are available in the art. Examples of useful algorithms are PILEUP (Higgins & Sharp, CABIOS 5:151 (1989), BLAST and BLAST 2.0 (Altschul et al. J. Mol. Biol. 215: 403 (1990). Software for performing BLAST analyses is publicly available through the National Center for Biotechnology Information (http://www.ncbi.nlm.nih.gov/).

In order to reconstruct the evolutionary relationships and sequence identity of one bacterial isolate to another, phylogenetic approaches are used standardly exploiting the 16S rRNA sequence or a portion of the 16S rRNA sequence of the bacteria, although any other sequence or the entire genome of the microorganisms to be analyzed can also be used. In microbiology, "16S rRNA sequence" refers to the sequence derived by characterizing the nucleotides that comprise the 16S ribosomal RNA gene(s). The bacterial 16S rRNA is approximately 1500 nucleotides in length.

The term "abundance" as used herein refers to the "amount" or "quantity" of particular taxa of gut microbiota, e.g. a bacterial genus or species that is relevant for inflammatory arthritis or that is relevant for the risk to develop joint and/or gut inflammation in a patient with inflammatory arthritis. In some embodiments, *Dialister* spp. are one of the said bacterial taxa. In other embodiments, the OTUs which are relevant for this application and are defined by their 16S rRNA sequence are presented in Table 4. According to current classification these OTUs relate to *Dialister invisus, D. propionifaciens, D. succinatiphilus, D. micraerophilus* and *D. pneumosintes.* Methods to measure the "abundance" of particular bacteria such as Dialister spp. are known to the skilled person. As example, without the purpose of limiting, the "abundance" of e.g. *Dialister* spp. can be based on analysis of amplification products of genes coding for one or more small subunit rRNA, etc. and/or based on analysis of proteins and/or metabolic products present in the biological sample, wherein the said genes, said proteins and/or said metabolic products are specific for *Dialister* spp.

The term "biological therapy" as used herein refers to a therapy that involves the use of living organisms, substances derived from living organisms, natural substances, artificial substances or laboratory-produced versions of such substances to stimulate or restore the ability of the body's immune system to fight infection and disease. Non-limiting examples are TNF-alpha blockers, anti-IL17A monoclonal antibodies, amongst others.

The term "disease activity score" as used herein refers to an assessment to measure disease activity of inflammatory arthritis and more particular of SpA, to determine whether the signs and symptoms have reduced or stopped, and if treatment needs to be adjusted. Progression or worsening of the disease will lead to an increased disease activity score. Non-limiting examples of disease activity scores for SpA patients are mentioned in the Background section. Also for rheumatoid arthritis, disease activity scores have been developed. A person skilled in the art is familiar with those. For rheumatoid arthritis, accurate reflections of disease activity include but are not limited to the Clinical Disease Activity Index, Disease Activity Score with 28-joint counts (erythrocyte sedimentation rate or C-reactive protein), Patient Activity Scale (PAS), PAS-II, Routine Assessment of Patient Index Data with 3 measures, and Simplified Disease Activity Index (Anderson et al. 2012 Rheumatoid arthritis disease activity measures: American college of rheumatology recommendations for use in clinical practice; Arthritis Care & Research 64, 640-647).

In one embodiment, the present application provides methods as defined by the appended claims.

The methods of the application may involve steps of identifying a patient, the health or medical condition of whom might benefit from the knowledge provided by the method. The patient may be asymptomatic at the time of the analysis, or the patient may be in the early, or even later, stages of the disease, and can benefit from the knowledge of the status of the gut microflora. In order to practice the methods of the application, generally a sample of gut microbiota is obtained from the patient by any method known to those of skill in the art, and the sample is tested for the presence or absence of, and/or for the relative abundance of, at least one taxon of bacteria. Generally, the taxa which are targeted for assessment are one or more taxa, the presence of which is known to be correlated with a particular disease or condition, or with particular symptoms associated or correlated with a disease/condition. In some embodiments, identification of a single or a few (e.g. about 10 or fewer, or about 100 or fewer) key microbes may be sufficient to link the presence of the microbes to the likely development of a disease. However, in other embodiments, a broad taxonomy determination is made, e.g. dozens, hundreds or thousands (or more) taxa may be targeted for assessment of their presence and/or absence and/or relative abundance.

Suitable biological samples for interrogation using the methods of the application include but are not limited to: samples of gut contents and/or mucosal biopsies obtained directly by an invasive technique e.g. by surgery, by rectal or intestinal sampling via colonoscopy-type procedures, or by other means. Preferably, samples are obtained by less invasive methods, e.g. stool samples, blood samples, serum samples, urine samples etc. In one embodiment, oral samples, such as oral rinses, oral swabs, sub gingival plaques etc. are collected e.g. to correlate the oral microbiome with the gut microbiome, or for other purposes. It is clear that the methods of current application do not include the sampling as such. Biological samples suitable and needed to practice the methods of the application are available before the methods are performed or executed.

After a sample is obtained, the types and/or the quantity (e.g. occurrence or abundance) in the sample of at least one bacteria of interest is determined according to any method known to those of skill in the art. In addition, a total amount of bacteria may be determined, and then for each constituent bacteria, a fractional percentage (e.g. relative amount, ratio, distribution, frequency, percentage, etc.) of the total is calculated. The result is typically correlated with at least one suitable control result, e.g. control results of the same parameter(s) obtained from asymptomatic individuals (negative control), and/or individuals known to have a disease of interest (positive control), or from subjects who have had the disease of interest and are being or have been treated, either successfully or unsuccessfully, etc.

If a strong correlation between a condition of interest and only one or a few microbes has previously been established, it is possible that detection of their presence (or absence) alone will suffice to justify or suggest a conclusion that the individual being tested does or does not have a high risk of developing the condition of interest. In this case, detection may be done in any of a number of ways that are known to those of ordinary skill in the art, including but not limited to culturing the organism or the few organisms, conducting various analyses which are indicative of the presence of the microbe(s) of interest (e.g. by microscopy, using staining techniques, enzyme assays, antibody assays, etc.), or by sequencing of genetic material (DNA or RNA), and others. However, generally it will be beneficial to obtain as much information as possible (or at least more information) regarding the microflora present in the sample. Older techniques (e.g. cultivation) are generally impractical for such an undertaking. Thus, newer nucleic acid sequencing technology (NextGen technology) is usually used. While any category (or categories) of nucleic acid(s) may be detected (usually amplified using, e.g. PCR techniques), particularly useful amplification strategies include the use of primers (e.g. universal primers) which amplify ribosomal RNA genes (rRNA). Such techniques and primers are well-known to those of skill in the art, e.g. see: Quince et al., "Accurate determination of microbial diversity from 454 pyrosequencing data", Nature methods 6.9 (2009): 639-641; Whiteley et al., "Microbial 16S rRNA Ion Tag and community metagenome sequencing using the Ion Torrent (PGM) Platform", Journal of microbiological methods 91.1 (2012): 80-88; Caporaso et al. 2012, "Ultra-high-throughput microbial community analysis on the Illumina HiSeq and MiSeq platforms", ISME J.; Kozich et al., "Development of a dual-index sequencing strategy and curation pipeline for analyzing amplicon sequence data on the MiSeq Illumina sequencing platform", Applied and environmental microbiology 79.17 (2013): 5112-5120;

In some embodiments, what is determined is the abundance of microbial families within the microbiome. However, characterization may be carried to more detailed levels, e.g. to the level of genus and/or species, and/or to the level of strain or variation (e.g. variants) within a species, if desired (including the presence or absence of various genetic elements such as genes, the presence or absence of plasmids, etc.). Alternatively, higher taxonomic designations can be used such as Phyla, Class, or Order. The objective is to identify which bacteria are present in the sample from the individual and the relative abundance of those microbes, e.g. expressed as a percentage of the total number of microbes that are present, thereby establishing a microbiome profile or signature for the individual being tested, e.g. for the region of the gut that has been sampled, or for the type of sample that is analyzed.

Once an individual patient's gut microbiome profile with respect to the targeted microbes has been determined, it is compared to known reference profiles obtained previously from control experiments. Such control experiments typically obtain "negative control" data from normal (healthy) individuals, i.e. comparable individuals who do not have disease symptoms, and positive control data from comparable individuals who do have the disease in question or particular disease symptoms or did have at the time of the analysis. Based on a comparative analysis between the patient's gut microbiome profile and one or more reference or control microbiome profiles (and usually corroborated statistically by methods that are well-known to those of ordinary skill in the art) the likelihood or risk of the patient for developing the disease or condition of interest is determined and thus can be used as a predictive diagnostic. For example, a person with a profile that is not similar to or within the range of values seen in normal control signatures, but which is more similar to or within ranges determined for positive controls, may be deemed to be at high risk for developing the disease. This is generally the case, for example, if his/her level or amount of at least one correlatable taxum is associated with the disease state with a statistically significant (P value) of less than about 0.05 (after multiple testing correction). Alternatively, for patients who are already symptomatic, a previous diagnosis may be corroborated, and/or an explanation of symptoms may be provided.

In other embodiments of the invention, when many taxa are being considered, the overall pattern of microflora is assessed, i.e. not only are particular taxa identified, but the percentage of each constituent taxon is taken in account, in comparison to all taxa that are detected and, usually, or optionally, to each other. Those of skill in the art will recognize that many possible ways of expressing or compiling such data exist, all of which are encompassed by the present invention, for example, the relationships may be expressed numerically or graphically as ratios or percentages of all taxa detected, etc. Further, the data may be manipulated so that only selected subsets of the taxa are considered (e.g. key indicators with strong positive correlations). Data may be expressed, e.g. as a percentage of the total number of microbes detected, or as a weight percentage, etc.

In one embodiment, a nonparametric multivariate test such as Metastats, Analysis of Similarity, Principle Component Analysis, Non-Parametric MANOVA (Kruskal-Wallace), or other tests as described in a non-limitative way in the Example section, can be used to associate microbiome dysbiosis with a statistical significant (P value) of less than 0.05 (after multiple testing correction). Such tests are known in the art and are described, for example, by White et al., "Statistical methods for detecting differentially abundant features in clinical metagenomic samples", PLoS Comput Biol 5.4 (2009): e1000352; Segata et al., "Metagenomic biomarker discovery and explanation", Genome Biol 12.6 (2011): R60.

In other embodiments, phylogenetic methods such as Unifrac can be used to associate microbiome dysbiosis with the disease state with a statistically significant (P value) of less than 0.05 (after multiple testing correction). See, for example, Lozupone C, Knight R (2005) UniFrac: a new phylogenetic method for comparing microbial communities. Appl Environ Microbiol 71:8228-8235.

In other embodiments, still other methods can be used to associate microbiome dysbiosis with the disease state with a statistically significant (P value) of less than 0.05 (after multiple testing correction). See for example: Linear models (MaAsLin - Tickle T, Waldron L, Yiren Lu, Huttenhower C. Multivariate association of microbial communities with rich metadata in high-dimensional studies, e.g. as in Morgan et al. Genome Biol 2015, 16:67); Machine Learning tools such as Random Forests, Support Vector Machines; ecological visualization tools (vegan: Community Ecology Package. R Package [Internet]. 2015 J Oksanen, F Blanchet, R Kindt, P Legendre, P Minchin, R O'Hara), amongst others.

Once a patient is identified as having, or at high risk for developing, the disease or condition as described herein, suitable clinical intervention can be undertaken to alter the identity and/or the relative abundance of gut microflora in the individual. Accordingly, the present invention also encompasses the identification of suitable therapeutic targets for intervention and the selection/development of suitable treatment protocols. Exemplary treatments include but are not limited to: eliminating or lessening microflora associated with the condition e.g. using antibiotics or other therapies, for example, therapies that are specific for eliminating or lessening the number of targeted microflora, without affecting or minimally affecting desirable microflora, if possible; or increasing microflora that compete with the unwanted microflora, and/or which are correlated with a lack of disease symptoms, e.g. by administering probiotic and/or prebiotic supplements; by microfloral transplants (e.g. from healthy donors); by dietary modifications; by lifestyle modifications; etc.

This is equivalent as saying that methods are provided for determining the presence gut inflammation in a patient with SpA comprising the steps of:
- determining a gut microbiome profile for said patient, wherein said gut microbiome profile comprising an indication of the presence and/or relative abundance of at least *Dialister spp.*;
- comparing said gut microbiome profile of said patient to one or more gut microbiome reference profiles, wherein said one or more gut microbiome reference profiles comprise at least one of a positive gut microbiome reference profile based on results from control subjects with gut inflammation and a negative gut microbiome reference profile based on results from control subjects without gut inflammation;
wherein a gut microbiome profile for said patient that statistically significantly matches said positive gut microbiome reference profile is indicative for said patient to have or to be at risk of developing gut inflammation.

The studies described herein have demonstrated that the presence and/or absence and/or abundance of certain genera of bacteria are associated with inflammatory arthritis patients, and more particularly with joint and/or gut inflammation in inflammatory arthritis patients, and more particularly with joint and/or gut inflammation in SpA patients. In exemplary embodiments, in mucosal and stool samples, bacteria such as *Dialister* are associated with disease activity in SpA patients. Thus, in a preferred embodiment, the gut microbiome profile of the SpA patient preferably comprises an indication of the presence and/or abundance of at least *Dialister* spp., including but limited to, *Dialister succinatiphilus, Dialister invisus,* amongst others. In another preferred embodiment, said at least *Dialister* spp. comprises one or more *Dialister* spp. that comprise a 16S rRNA sequence with an at least 95%, at least 96%, at least 97%, at least 98%, at least 99%, at least 99.5%, at least 100% identity to a 16S rRNA sequence selected from Table 4. In another preferred embodiment, said at least *Dialister* spp. comprises one or more *Dialister succinatiphilus, Dialister invisus, Dialister propionifaciens, Dialister micraerophilus* or *Dialister pneumosintes* or combinations thereof. In another preferred embodiment, said at least *Dialister* spp. comprises one or more *Dialister* OTUs as listed in Table 4. In another preferred embodiment, said at least *Dialister* spp. comprises at least OTU44, OTU60 or OTU62 as specified in Table 4.

Further embodiments of the invention provide methods for determining reference gut microbiome profiles, and databases comprising the same. Such profiles constitute prototypes or models for use as references when the assessment of an individual's microflora is undertaken. In some embodiments, control signatures are collected and averaged or amalgamated to develop reference profiles which are correlated with a disease or condition of interest (and/or with the absence of the disease/condition). The reference profiles may be in the form of e.g. sequences which are characteristic of particular bacterial types, according to any useful classification (phylum, order, class, genus, species, strain, type, etc.). Further, the reference profiles generally include this information for relevant groups and subgroups of microflora, e.g. those associated with a particular disease, condition, etc. The characteristics of the reference profiles are generally recorded (stored, compiled, etc.) in an electronic computerized catalog, library, database, etc. that is accessible to a practitioner of the invention. Such databases may include Genbank, the Ribosomal Database, Greengenes, Silva, amongst others. The invention also encompasses computer programs (e.g. executable software programs, and/or computers configured to carry out the programs), which enable a practitioner to enter analytical data into the system (e.g. the results of rRNA PCR amplification of a stool sample, which may be the patient's gut microbiome profile) and to carry out a comparison to the stored reference profiles. Output from the program may include an expression of the level of similarity between the patient's signature and one or more relevant stored reference profiles, and/or the statistical likelihood that the patient already has or is likely to develop a disease or condition associated with one or more reference profiles.

In some embodiments, the gut microbiome profile of a subject includes (or is) the result(s) of an analysis of the metabolome of the subject. In other words, instead of, or in addition to, determining the identity, the presence or absence, and/or abundance of bacteria in the gut, the identity, presence or absence and/or abundance of selected bacterial metabolic products of interest is determined. Exemplary metabolites include but are not limited to indoles, short chain fatty acids, amino acids, bile acids, etc. The metabolites may be associated with (e.g. characteristic of) one or more bacterial taxa of interest. Exemplary metabolites that may be included in such a gut metabolome signature include but are not limited to volatile organic compounds detected by GC-MS, and hydrophobic and hydrophilic organic compounds detected by LC-MS. In some embodiments, nuclear magnetic resonance (NMR) is utilized for detection. Other detectable metabolites are known to those of skill in the art.

In yet other embodiments of the application, the gut microbiome profile of a subject may be, or may include, results obtained by analyzing the protein content of a biological sample (e.g. a gut sample), of a subject. The results may include the identity of the proteins, the presence or absence of selected proteins, the abundance of the proteins (e.g. compared to suitable controls), etc. The proteins may be associated with (e.g. characteristic of) one or more bacterial taxa of interest. Exemplary proteins that may be included in such a gut proteome profile include but are not limited to those which are known to those of skill in the art.

In a particular embodiment, said *Dialister* spp. is a *Dialister* spp. that comprises a 16S rRNA sequence with an at least 95%, at least 96%, at least 97%, at least 98%, at least 99%, at least 99.5%, at least 100% identity to one of the 16S rRNA sequences listed in Table 4. In a more particular embodiment, said *Dialister* spp. is *Dialister succinatiphilus, Dialister invisus, Dialister propionifaciens, Dialister micraerophilus* or *Dialister pneumosintes.* In an even more particular embodiment, said *Dialister* spp. is a *Dialister* OTU listed in Table 4. In an even more particular embodiment, said *Dialister* spp. is OTU44, OTU60 or OTU62 as listed in Table 4.

Preferably, the abundance of a *Dialister* spp. is determined by 16S rRNA sequencing as explained above. However, detecting the presence and determining the abundance can also be performed indirectly by determining metabolic products or proteins which are produced specifically by said *Dialister* spp.

In a particular alternative of all above embodiments and provided methods, said inflammatory arthritis is spondyloarthritis.

Preferably, a biological therapy is used, and includes, but is not limited to, TNF-alpha blockers, anti-IL17A monoclonal antibodies, amongst others.

In a particular alternative of above embodiments and provided methods, said inflammatory arthritis is spondyloarthritis.

Another aspect of the application is to provide methods for monitoring the efficacy of a treatment that is treating a condition or complication associated with spondyloarthritis. The method involves determining gut microbiome profiles of a patient who is or who is going to be treated for SpA for a condition or complication of interest associated with SpA e.g. gut inflammation. Multiple profiles are generally obtained and analyzed at suitable time intervals, e.g. just prior to treatment to establish a baseline, and then repeatedly every few days, weeks or months thereafter. Subsequent profiles are compared to suitable reference profiles and/or to one or more previous profiles from the patient. If subsequent profiles indicate that the patient's gut microbiome profile is improving (e.g. is more similar to that of controls who do not have the condition of interest, especially when compared to previous patient signatures) then the treatment may be continued without adjustment, or may be gradually decreased, and may even be discontinued. However, if no improvement is observed, or if a signature indicates a worsening of the condition, then the treatment protocol can be adjusted accordingly, e.g. more of a treatment agent may be administered, or a different and/or more drastic form of treatment may be implemented, etc. The gut microbiome profile is thus used to assess treatment adequacy and treatment response after therapies such as those available for inflammatory arthritis or more particularly spondyloarthritis.

In another embodiment, the application also provides methods to monitor the efficacy of a treatment for gut inflammation in a patient with a SpA disease, said method comprising the steps of:
- determining a gut microbiome profile for said patient, said gut microbiome profile comprising an indication of the presence and/or relative abundance of at least *Dialister* spp.;
- comparing said gut microbiome profile of said patient to one or more gut microbiome reference profiles, wherein said one or more gut microbiome reference profiles comprise one or more of a positive gut microbiome reference profile based on results from control subjects with gut inflammation and a negative gut microbiome reference profile based on results from control subjects without gut inflammation;

wherein a gut microbiome profile for said patient that statistically significantly matches said negative gut microbiome reference profile is indicative for an efficacious treatment; and
wherein said analyzing and comparing steps are performed at one or more successive time points with biological samples collected from said patient at one or more successive time periods during said treatment.

In another embodiment, a method is provided to monitor the efficacy of a treatment for gut inflammation in a patient with a SpA disease, said method comprising the steps of:
- determining the abundance of a *Dialister* spp. in a biological sample of said patient at two or more successive time points with biological samples collected from said patient at two or more successive time periods during said treatment;
- if the abundance of said *Dialister* spp. for said patient at a later time point of said two or more successive time points is at least 10%, at least 20%, at least 30%, at least 40%, at least 50%, at least 60%, at least 70%, at least 80%, at least 90%, at least 95% lower than the abundance of said *Dialister* spp. at an earlier time point of said two or more successive time points, then concluding that said treatment is efficacious.

Preferably, a biological therapy is used, and includes, but is not limited to, TNF-alpha blockers, anti-IL17A monoclonal antibodies, amongst others.

Another aspect of the application is the use of a gut microbiome profile to detect the presence of gut inflammation in a patient with a SpA disease, wherein said gut microbiome profile comprises an indication of the presence and/or abundance of at least *Dialister* spp.

In a particular extension of all embodiments of above described aspects and of all embodiments in current application, said at least *Dialister* spp. comprises one or more *Dialister* spp. that comprise a 16S rRNA sequence with an at least 95%, at least 96%, at least 97%, at least 98%, at least 99%, at least 99.5%, at least 100% identity to a 16S rRNA sequence selected from Table 4. In another particular extension of all embodiments of above described aspects and of all embodiments in current application, said at least *Dialister* spp. comprises at least *Dialister succinatiphilus, Dialister invisus, Dialister propionifaciens, Dialister micraerophilus* or *Dialister pneumosintes.* In another particular extension of all embodiments of above described aspects and of all embodiments in current application, said at least *Dialister* spp. comprises one or more *Dialister* OTUs listed in Table 4. In another particular extension of all embodiments of above described aspects and of all embodiments in current application, said at least *Dialister* spp. comprises at least OTU44, OTU60 or OTU62 as specified in Table 4.

In a particular extension of all embodiments of above described aspects and of all embodiments in current application, said *Dialister* spp. is a *Dialister* spp. that comprises a 16S rRNA sequence with an at least 95%, at least 96%, at least 97%, at least 98%, at least 99%, at least 99.5%, at least 100% identity to one of the 16S rRNA sequences listed in Table 4. In a more particular extension of all embodiments of above described aspects and of all embodiments in current application, said *Dialister* spp. is *Dialister succinatiphilus, Dialister invisus, Dialister propionifaciens, Dialister micraerophilus* or *Dialister pneumosintes.* In an even more particular extension of all embodiments of above described aspects and of all embodiments in current application, said *Dialister* spp. is a *Dialister* OTU listed in Table 4. In an even more particular extension of all embodiments of above described aspects and of all embodiments in current application, said *Dialister* spp. is OTU44, OTU60 or OTU62 as specified in Table 4.

In a most particular extension of all embodiments of above described aspects and embodiments in current application, said gut inflammation is not Crohn's disease.

The following examples are intended to promote a further understanding of the invention. While the invention is described herein with reference to illustrated embodiments, it should be understood that the invention is not limited hereto. Those having ordinary skill in the art and access to the teachings herein will recognize additional modifications and embodiments within the scope thereof. Therefore, the invention is limited only by the claims attached herein.

### EXAMPLES

### Example 1. Patient characteristics for biopsies

Mucosal samples from 27 SpA patients and 15 healthy controls were used. Patient characteristics at baseline according to bowel histology are summarized in Table 1. Consistent with earlier reports, chronic inflammation was associated with younger age and male sex (p= 0.010 and p = 0.021, respectively). None of the other clinical characteristics (BMI, symptom duration, CRP, BASDAI, ASDAS, HLAB27, AS versus non-radiographic axial SpA, smoking status) were significantly associated with bowel histology. There was also no significant difference in NSAID index.

### Example 2. Microbial composition and diversity across biopsy samples

We first assessed overall variation of microbial composition and diversity across samples. Microbial diversity comprises richness (observed species; here measured by observed OTU richness, Figure 1a) and evenness (how the relative abundance or biomass is distributed among species); these combined properties are measured by the Shannon Diversity index, Figure 1b. Whole sample community composition-based ordination on Bray Curtis distances did not show clear sample separation based on biopsy location (Figure 1c). Likewise no significant differences in richness and evenness of colonic and ileal biopsies were detected. Overall, bacterial profiles of ileum and colon were more similar within one individual than same-site samples across persons. However, all further analyses were performed on either ileal or colonic samples, using only one biopsy sample per patient. Given the overall low bacterial yield from biopsy samples, we next assessed any interference from contaminant bacteria. To this aim, we analysed the bacterial profile of negative controls (blanks; see methods). Negative controls exhibit a profile dominated by genera belonging to the phylum Proteobacteria, which represents a minor contributor to the profile of our biopsies. The profile of gut microbiota reported in this study is consistent with the general profile of the human gut microbiome, dominated by Firmicutes and Bacteroidetes. These results rule out any major contamination event during the generation of molecular data.

### Example 3. Intestinal inflammation versus microbial diversity and composition in biopsy samples

Inflamed ileum and colon biopsies presented significantly higher Shannon Diversity Index (evenness) compared to non-inflamed samples (ileum p=0.030 and colon p=0.025). Also, a trend towards greater microbial richness was observed in inflamed versus non-inflamed biopsies (Figure 2 + Figure 8). Microbial richness was higher in chronically inflamed versus acute or non-inflamed biopsies. After performing non-parametric Welch Two Sample t-test with 1000 permutations and multiple testing corrections, a significant difference in richness between acute and chronically inflamed ileal biopsies was observed (p=0.009, FDR-corrected q-value of 0.027). Likewise, for ileal biopsies a trend of positive association between the type of inflammation and microbial evenness was found, with higher evenness in acutely inflamed biopsies, followed by chronic inflamed biopsies and lower levels in non-inflamed and healthy control biopsies (Figure 2 + Figure 8). We also assessed the effect of systemic inflammation on microbial diversity, but found no significant link between CRP levels and diversity measurements. Next, we assessed whether overall bacterial community composition was associated with gut histology. A PCoA on Bray-Curtis dissimilarity showed mild separation by inflammation status, which was confirmed by permutational multivariate analysis of variance (PERMANOVA) (p-value of 0.016 and 0.011 for ileum and colon respectively; Figure 3 and 4). Hierarchical clustering on Bray-Curtis distances revealed that inflamed ileal biopsies (acute and chronic) formed a cluster separated from non-inflamed biopsies (Figure 3). Observations in colonic biopsies were highly consistent with those in the ileal biopsies (Figure 4). In conclusion, our results indicate a significant difference in intestinal microbial composition in SpA patients with and without microscopic gut inflammation.

### Example 4. Dialister, a potential microbial marker of disease activity in SpA patients

To further explore the relationship between bacterial composition and inflammation, we tested which bacterial genera were significantly associated with gut histology. However, no specific genus was significantly correlated with gut inflammation (Table 2 and 3). Next, we examined a possible link between bacterial composition and disease activity parameters. We observed a positive correlation between the abundance of the genus *Dialister* and ASDAS (FDR-corrected q-value of 0.026) (Figure 5a; results are given for ileal biopsies). A positive correlation between *Dialister* and BASDAI and CRP was also found. Results were consistent between acute and chronic inflammation, but the association between *Dialister* and ASDAS was most pronounced in chronically inflamed biopsies (Figure 5b). In colonic biopsies, a consistent correlation was observed (Figure 6) as expected given the high concordance between *Dialister* abundances in colonic and ileal samples (Figure 7). (Nonparametric) t-test analysis revealed that abundance of *Dialister* was significantly higher in ileal and colonic biopsies from the inflamed versus non-inflamed and healthy control groups (FDR < 0.10), with higher abundance seen in acutely inflamed biopsies (Figure 9). More precisely, for ileal biopsies, we observed a 3.5 fold increase in the abundance of *Dialister* in inflamed biopsies from SpA patients compared to healthy controls and a 1.3 fold increase in the abundance of *Dialister* in non-inflamed biopsies from SpA patients compared to healthy controls. Still for ileal biopsies, we observed a 5.1, 2.6 and 1.3 fold increase in the abundance of *Dialister* in acute, chronic and non-inflamed biopsies respectively from SpA patients compared to healthy controls. For colonic biopsies, we observed a 6.8 and 1.9 fold increase in abundance of *Dialister* in inflamed and non-inflamed biopsies respectively from SpA patients compared to healthy controls. For colonic biopsies, we also observed a 5.8, 6.8 and 1.9 fold increase in the abundance of *Dialister* in acute chronic and non-inflamed biopsies respectively from SpA patients compared to healthy controls. We also found a trend for higher levels of specific genera that have previously been reported to be associated with inflammation (i.e. *Prevotella* (Scher et al. 2013, eLife;2:e01202) in the inflamed (chronic and acute) biopsies. In conclusion, we identified *Dialister* as a potential microbial marker of disease activity.

To further understand the *Dialister* microbiome composition, we performed a phylogenetic study of several *Dialister* OTUs (operational taxonomic units) which are high-abundant in biopsy samples of SpA patients or in stool samples of persons that volunteered in the Flemish Gut Flora project (FGFP). More precisely, a phylogenetic analysis was performed using the maximum likelihood method based on partial 16S rRNA gene sequences encompassing 250 positions corresponding to the V4-16S rDNA region, thus covering app. 15% of the 16S rRNA gene. The FGFP is a large-scale cross-sectional fecal sampling effort in a confined geographical region, more precisely Flanders (Belgium). The FGFP cohort is expected to be representative for the average gut microbiota composition in a Western European population (Falony et al 2016 Science 352: 560-564). Surprisingly, a clear difference was found between SpA samples and FGFP samples. OTU60 and OTU62 were the most abundant *Dialister* OTUs in SpA patients. Both OTUs had a 100% identical nucleotide sequence (based on 250 positions of V4-16S rDNA) to *D. invisus.* The most abundant *Dialister* OTUs in FGFP samples were OTU4552 that shows highest similarity to *D*. *micraerophilus,* OTU6938 that shows highest similarity to *D. succinatiphilus,* OTU1378 that does not show high similarity to a known *Dialister* species, and OTU605 and OTU233 that both show highest similarity to *D. invisus.*

### Example 5. Dialister OTU distribution in stool samples of SpA patients

Following up on the *Dialister* results regarding colonic and ileal mucosa, we examined stool samples from SpA patients with different levels of ASDAS (Ankylosing Spondylitis Disease Activity Score), NSAID (Nonsteroidal anti-inflammatory drug), BASDAI (Bath Ankylosing Spondylitis Disease Activity Index), Inflammation activity and fecal calprotectin. Characteristics of the SpA patients are summarized in Table 5. Bacteria profiling from 46 stools samples was characterized using the methods described in this application. Sequences were quality trimmed, demultiplexed, analyzed and taxonomy identified against RDP/Greengenes database using LotuS. All reads per sample were used for *Dialister* abundance analysis. The rest of the analysis was performed using R libraries on data rarefied at 10000 observations/reads per sample. Using all reads, *Dialister* is present in 74% of the stools samples from SpA patients (35 of 46 patients), with an abundance ranging from 3.3E-17 to 7.7% (Figure 10). From the 16 different OTUs assigned to the genus Dialister, OTU 44 is the main contributor, followed by OTU 515, OTU 420 and OTU 685. Similar to the most abundant OTU in biopsy samples (see Example 4), OTU 44 exhibits a sequence match of 100% to *Dialister invisus.* The other OTUs cannot be reliably assigned to a known *Dialister* species based on what is available so far in NCBI.

### Example 6. Correlation between Dialister abundance in stool samples and ASDAS

The studied stool samples (n=46 stool samples from patients with SpA) exhibit a positive correlation between *Dialister* and ASDAS. This correlation is improved when samples are grouped by gut inflammation status (normal vs. inflamed biopsies). The positive correlation value of 0.13 for all stool samples can be divided in 0.29 for patients with inflammation and -0.04 for patients without inflammation. This pattern is consistent with our results of *Dialister* abundance in colonic and ileal mucosa, but it is not significant (Figure 11). The correlation is driven by OTU 44, which is the most abundant OTU assigned to the *Dialister* genus (Figure 10).

### Example 7. Correlation between Dialister abundance in stool samples and NSAID

*Dialister* abundance positively correlates with levels of intake of non-steroidal anti-inflammatory drugs (Figure 12, rho=0.34, p< 0.01), thus further building evidence for a clear link between Dialister abundance and SpA. In conclusion, the data presented in Examples 5-7 clearly show that the positive correlation between *Dialister* abundance and SpA can also be observed in stool samples. These surprising findings are of great importance for the diagnosis of SpA and other inflammatory arthritis diseases, since these data show that inflammatory arthritis disease can be detected or diagnose using non-invasive sampling techniques.

### Example 8. Dialister abundance in oral samples is correlated with SpA

Bisanz et al, 2016 (The oral microbiome of patients with axial spondyloarthritis compared to healthy individuals; Peer J 4:e2095) compares the oral microbiome of patients with axial spoondyloarthritis (AxSpA) with healthy individuals and did not find strong evidence of any single taxa associated with AxSpA in the sub gingival plaque. After performing reanalysis of the data, and looking for genera enriched or depleted in these two groups, we confirmed their finding, not finding significant differences in the abundance between groups. However, the AxSpA group exhibits a higher mean abundance of *Dialister,* consistent with our findings in stool samples in the colonic and ileal mucosa (Figure 13). These data thus further broaden the scope of samples that can be used to diagnose inflammatory arthritis diseases in a patient. Beside invasive techniques such as biopsy, also samples obtained by non-invasive sampling techniques (stool, oral plaques) can be used.

### METHODS TO THE EXAMPLES

### Study Population and Sample Collection

Mucosal samples from 27 SpA patients (Table 1) and 15 healthy controls were used. This study was conducted after approval by the ethical committee of Ghent University Hospital and written informed consent was obtained from all patients. Patients with overt IBD were excluded from this analysis, as were patients with prior exposure to biologic therapy, or use of conventional DMARDs (sulfasalazine, methotrexate, leflunomide), corticosteroids or antibiotics 2 months prior to ileocolonoscopy. NSAID intake 2 months prior to the ileocolonoscopy was estimated by calculation of the NSAID index score, as recommended by ASAS (Dougados et al. 2011, Ann Rheum Dis 70:249-251). Ileal and colonic biopsies were classified as normal or inflamed (acute/chronic) by an experienced pathologist. Biopsies used for nucleic acid extraction were kept at -80°C in sterile cryovials.

Stool samples from 46 SpA patients (Table 5) were used. Patients were interviewed about their disease activity, drug intake and possible GI symptoms. A complete clinical examination was performed with scoring of tender and swollen joints, enthesitis and evaluation of axial mobility. Stool samples were collected within a week before colonoscopy (but prior to bowel preparation) and were stored unprocessed at -80°C. At a later stage, 1g aliquots were prepared. Patients with significant GI symptoms or overt IBD were excluded from this analysis. Non-steroidal anti-inflammatory drug (NSAID) intake 3 months prior to the ileocolonoscopy was estimated by calculation of the NSAID index score, as proposed by the Assessment of SpondyloArthritis international Society (ASAS).

### Nucleic acid extraction

A total of 54 biopsy samples, with associated clinical information, (27 colonic and 27 ileal) from SpA patients and 21 biopsy samples from healthy controls (15 ileal and 6 colonic) and a total of 46 stool samples, with associated clinical information from SpA patients were used for this study (Table 1 and 5). DNA was extracted from biopsies and stool samples using the PowerMicrobiome^{®} RNA Isolation Kit (MO BIO Laboratories Inc., Carlsbad, CA) following manufacturer's instructions, with the addition of 10 minutes at 90°C before vortexing and with the exclusion of the DNase I step. No more than 11 samples were prepared at the time and each set of extractions included a negative control (blank). All steps were performed under a biohazard type II cabinet and all material was decontaminated using UVC light. Surfaces and gloves were decontaminated using RNase AWAY^{®} (Molecular Bio-Products Inc., San Diego, CA).

### 16S rRNA profiling and analysis

To amplify the variable region 4 (V4) of the 16S rRNA gene, we used the 515F and 806R primers (GTGCCAGCMGCCGCGGTAA (SEQ ID NO. 25) and GGACTACHVGGGTWTCTAAT (SEQ ID NO. 26) respectively) modified to contain Illumina adapters and barcode sequences to allow for directional sequencing (Caporaso et al. 2011, Proc Natl Acad Sci USA, 108 Suppl 1:4516-4522). Sequencing was performed on the Illumina MiSeq platform (MiSeq Reagent Kit v2, 500-cycles, 20% PhiX) according to the manufacturer's specifications to generate paired-end reads of 250 bases in length in each direction. The overlapping paired-end reads were merged using fastq-join (Aronesty et al. 2013, Open Bioinforma J 7:1-8) and processed with LotuS pipeline (Hildebrand et al. 2014, Microbiome 2:30), clustering sequences into OTUs using Greengenes 2013 database for taxonomy assignation. Further analysis was performed in a subset of the data rarefied at 25000 and 20000 observations per sample using phyloseq (McMurdie et al. 2012, Pacific Symposium on Biocomputing 235-246) and R scripts.

### Statistical Analysis

Statistical significance was evaluated using non-parametric Welch Two Sample t-test with 1000 permutations, Mann-Whitney-Wilcoxon Test, Kruskal-Wallis H-test (non-parametric ANOVA) and Spearman rank correlation tests as specified throughout the results. The significance threshold was set at 0.05. Permutational Multivariate analysis of variance (PERMANOVA) was conducted with the vegan functions adonis using 10000 permutations. Correction for multiple testing (q-values) was performed by applying the Benjamini-Hochberg False Discovery Rate (FDR) approach. PERMANOVA and Kruskal-Wallis H-test were performed on filtered OTUs matrices for ileum and colon after removing OTUs with absolute abundance lower than 0.01%.

### TABLES

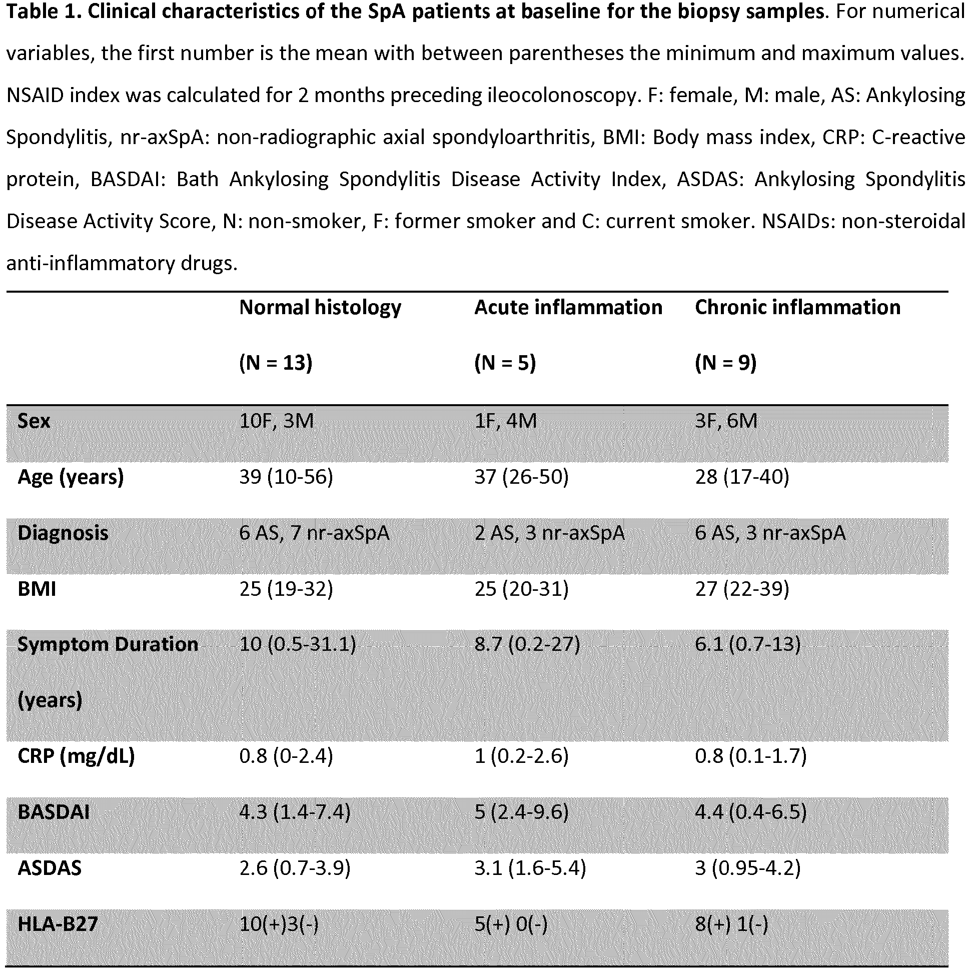

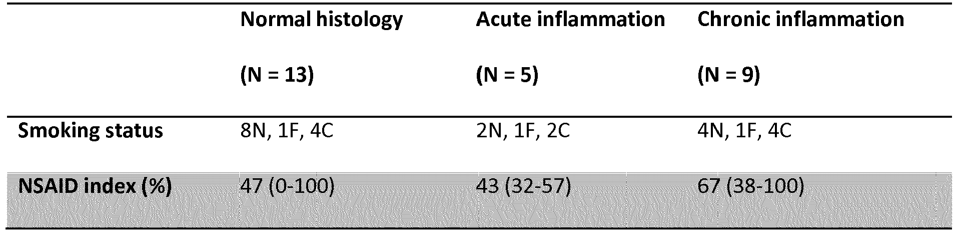

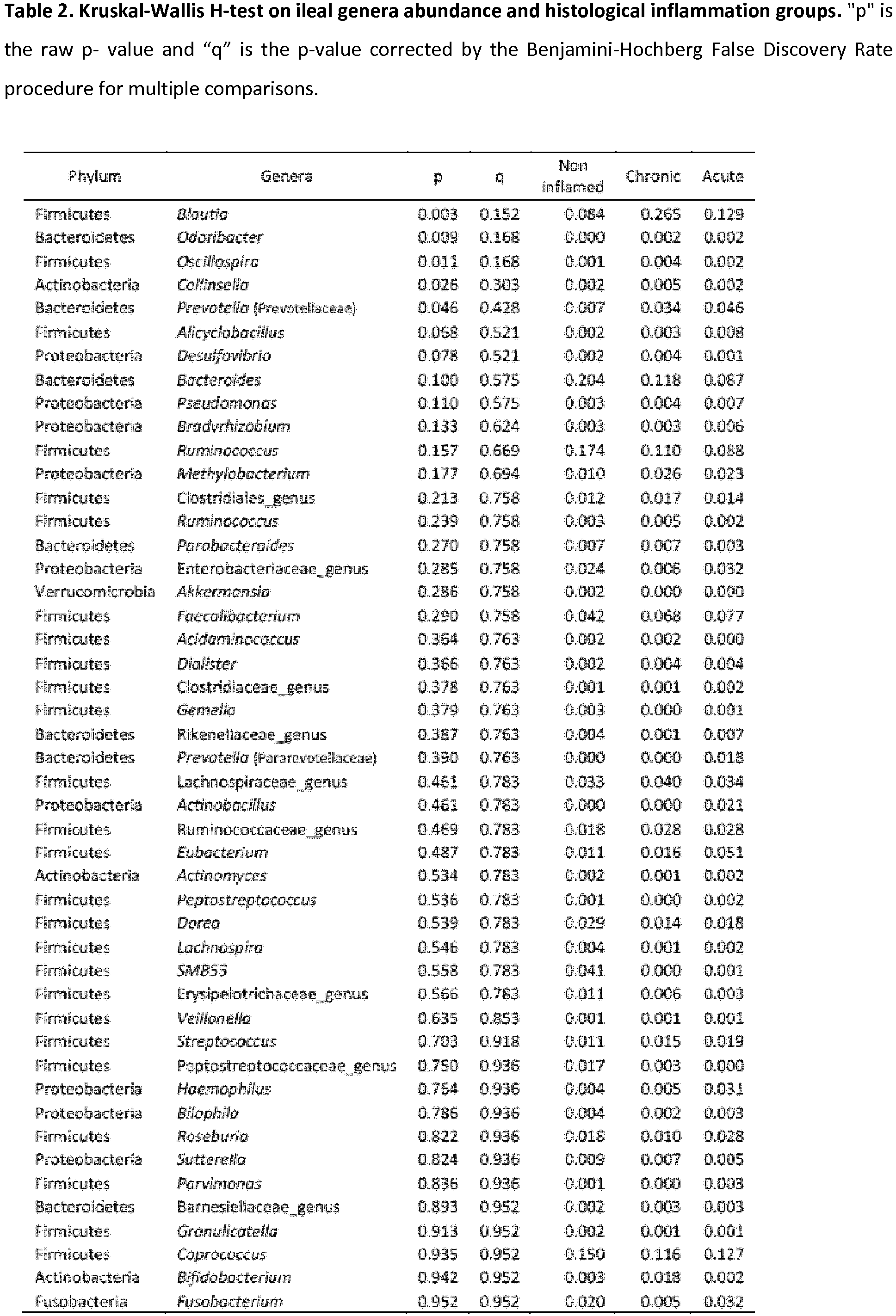

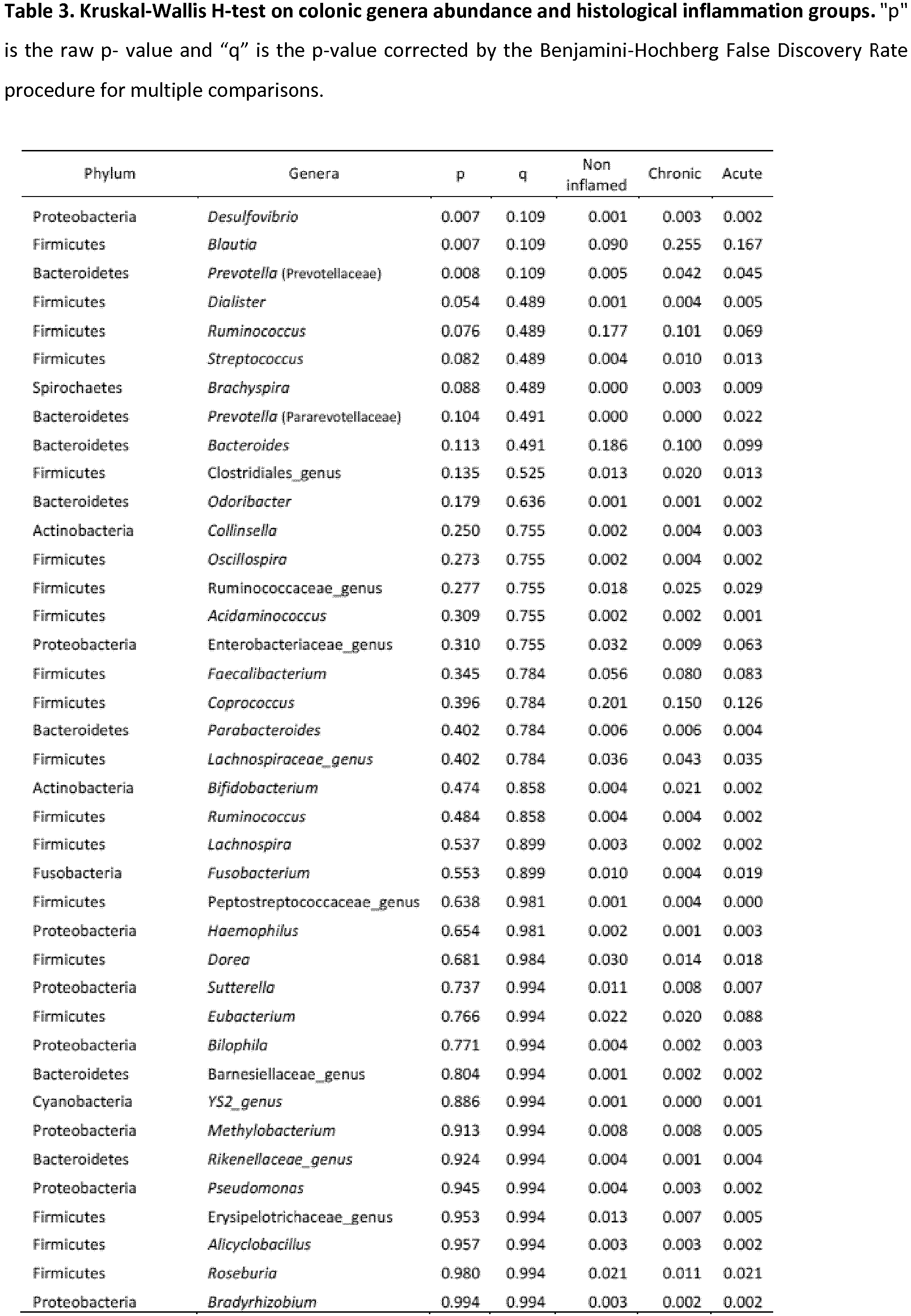

**Table 4. A selection of Dialister OTU's and their corresponding 16S rRNA fragments.**

| **Dialister OTU** | **Highest similarity to** | **16S rRNA fragment** | **SEQ ID NO.** |
|---|---|---|---|
| #OTU4552 | *D. micraerophilus* | TACGTAGGTGGCAAGCGTTGTCCGGAATTATTGGGCG TAAAGCGCGCGCAGGCGGCTTCTTAAGTCCATCTTAA AAGTGCGGGGCTTAACCCCGTGATGGGATGGAAACT GGGAGGCTGGAGTATCGGAGAGGAAAGTGGAATTCC TAGTGTAGCGGTGAAATGCGTAGAGATTAGGAAGAA CACCGGTGGCGAAGGCGACTTTCTGGACGACAACTGA CGCTGAGGCGCGAAAGCGTGGGGAGCAAACAGG | 1 |
| #OTU6938 | *D. succinatiphilus* | TACGTAGGTGGCAAGCGTTGTCCGGAATTATTGGGCG TAAAGCGCGCGCAGGCGGCTTTCTAAGTCCATCTTAA AAGTGCGGGGCTTAACCCCGTGATGGGATGGAAACT GGAAAGCTGGAGTATCGGAGAGGAAAGTGGAATTCC TAGTGTAGCGGTGAAATGCGTAGAGATTAGGAAGAA CACCGGTGGCGAAGGCGACTTTCTGGACGACAACTGA CGTTGAGGCACGAAAGCGTGGGTATCGAACAGG | 2 |
| #OTU1378 | Uncultured Dialister | TACGTATGGTGCAAGCGTTGTCCGGAATTATTGGGCG TAAAGCGCGCGCAGGCGGCTTTCTAAGTCCATCTTAA AAGTGCGGGGCTTAACCCCGTGATGGGAAGGAAACT GGGAAGCTGGAGTATCGGAGAGGAAAGTGGAATTCC TAGTGTAGCGGTGAAATGCGTAGAGATTAGGAAGAA CACCGGTGGCGAAGGCGACTTTCTGGACGAAAACTG ACGCTGAGGCGCGAAAGCGTGGGGAGCAAACAGG | 3 |
| #OTU605 | *Dialister invisus* | AACGTAGGTCACAAGCGTTGTCCGGAATTATTGGGCG TAAAGCGCGCGCAGGCGGCTTCCCAAGTCCCTCTTAA AAGTGCGGGGCTTAACCCCGTGATGGGAAGGAAACT GGGAAGCTGGAGTATCGGAGAGGAAAGTGGAATTCC TAGTGTAGCGGTGAAATGCGTAGAGATTAGGAAGAA CACCGGTGGCGAAGGCGACTTTCTGGACGAAAACTG ACGCTGAGGCGCGAAAGCGTGGGG-AGCAAACAGG | 4 |
| #OTU233 | *Dialister invisus* | TACGTAGGTGGCAAGCGTTGTCCGGAATTATTGGGCG TAAAGCGCGCGCAGGCGGCTTCCCAAGTCCCTCTTAA AAGTGCGGGGCTTAACCCCGTGATGGGAAGGAAACT GGGAAGCTGGAGTATCGGAGAGGAAAGTGGAATTCC TAGTGTAGCGGTGAAATGCGTAGAGATTAGGAAGAA CACCGGTGGCGAAGGCGACTTTCTGGACGAAAACTG ACGCTGAGGCGCGAAAGTGCGGGTATCGAACAGG | 5 |
| #OTU62 | *Dialister invisus* | AGTGTCAGCCGCCGCGGTAATACGTAGGTGGCAAGC GTTGTCCGGAATTATTGGGCGTAAAGCGCGCGCAGG CGGCTTCCCAAGTCCCTCTTAAAAGTGCGGGGCTTAA CCCCGTGATGGGAAGGAAACTGGGAAGCTGGAGTAT CGGAGAGGAAAGTGGAATTCCTAGTGTAGCGGTGAA ATGCGTAGAGATTAGGAAGAACACCGGTGGCGAAGG CGACTTTCTGGACGAAAACTGACGCTGAGGCGCGAAA GCGTGGGGAGCAAACAGG | 6 |
| #OTU60 | *Dialister invisus* | ACGTAGGTGGCAAGCGTTGTCCGGAATTATTGGGCGT AAAGCGCGCGCAGGCGGCTTCCCAAGTCCCTCTTAAA | 7 |
| | | AGTGCGGGGCTTAACCCCGTGAGGGAAGGAAACTGG GAAGCTGGAGTATCGGAGAGGAAAGTGGAATTCCTA GTGTAGCGGTGAAATGCGTAGAGATTAGGAAGAACA CCGGTGGCGAAGGCGACTTTCTGGACGAAAACTGAC GCTGAGGCGCGAAAGCGTGGGGAGCAAACAG | |
| #OTU373 | Uncultured Dialister | ACGTAGGTGGCAAGCGTTGTCCGGAATTATTGGGCGT AAAGCGCGCGCAGGCGGCTTCCCAAGTCCCTCTTAAA AGTGCGGGGCTTAACCCCGTGATGGGAAGGAAACTG GGAAGCTGGAGTATCGGAGAGGAAAGTGGAATTCCT AGTGTAGCGGTGAAATGCGTAGATATTAGGAGGAAC ACCAGTGGCGAAGGCGGCTTACTGGACAGTAACTGA CGTTGAGGCTCGAAAGCGTGGGGAGCAAACAG | 8 |
| #OTU_1251 | *D. invisus* | CCTGTTTGCTCCCCACGCTTTCGTGCCTCAGTGTCAGT AGCAGTCCAGTAAGTCGCCTTCGCCACTGGTGTTCCTC CTAATATCTACGCATTTCACCGCTACACTAGGAATTCC ACTTACCTCTCCGATACTCCAGCTTCCCAGTTTCCTTCC CATCACGGGGTTAAGCCCCGCACTTTTAAGAGGGACT TGGGAAGCCGCCTGCGCGCGCTTTACGCCCAATAATT CCGGACAACGCTTGCCACCTACGTA | 9 |
| # OTU_1303 | *D. propionifaciens* | CCTGTTTGCTCCCCACGCTTTCGTGCCTCAGTGTCAGT AGCAGTCCAGTAAGTCGCCTTCGCCACTGGTGTTCTTC CTAATCTCTACGCATTTCACCGCTACACTAGGAATTCC ACTTTCCTCTCCGATACTCCAGCCTCTCAGTTTCCATCC CATCACGGGGTTAAGCCCCGCACTTTTAAGATGGACT TAAGAAGCCGCCTGCGCGCGCTTTACGCCCAATAATT CCGGACAACGCTTGCCACCTACGTA | 10 |
| # OTU_1459 | Uncultured Dialister | CCTGTTTGCTCCCCACGCTTTCGCGCCTCAGCGTCAGT TTTCGTCCAGAAAGTCGCCTTCGCCACCGGTGTTCTTC CTAATATCTACGCATTTCACCGCTACACTAGGAATTCC ACCTTCCTCTCCGATACTCCAGTCTCCCAGTTTCCATCC CCTCACGGGGTTAAGCCCCGCACTTCTAAGATAGACT TAAGAGACCGCCTGCGCGCGCTTTACGCCCAATAATT CCGGACAACGCTTGCCACCTACGTA | 11 |
| # OTU_2237 | *D. invisus* | CCTGTTCGCTCCCCTAGCTTTCGCGCCTCAGCGTCAGT GGCGGCCCAGAAGGCTGCCTTCGCCATCGGTGTTCTT CCCAATATCTGCGCATTTCACCGCTACACTGGGAATTC CACTTTCCTCTCCGATACTCCAGCTTCCCAGTTTCCTTC CCATCACGGGGTTAAGCCCCGCACTTTTAAGAGGGAC TTGGGAAGCCGCCTGCGCGCGCTTTACGCCCAATAAT TCCGGACAACGCTTGCCACCTACGTA | 12 |
| # OTU_2284 | *D. invisus* | CCTGTTTGATACCCACACTTTCGAGCCTCAATGTCAGT TGCAGCTTAGCAGGCTGCCTTCGCAATCGGAGTTCTTC GTGATATCTAAGCATTTCACCGCTACACTAGGAATTCC ACTTTCCTCTCCGATACTCCAGCTTCCCAGTTTCCTTCC CATCACGGGGTTAAGCCCCGCACTTTTAAGAGGGACT TGGGAAGCCGCCTGCGCGCGCTTTACGCCCAATAATT CCGGACAACGCTTGCCACCTACGTA | 13 |
| # OTU_2460 | *D. invisus* | CCTGTTTGCTCCCCACACTTTCGCGCCTCAGCGTCAGT TACAGTCCAGTTAGTCGCCTTCGCCACTGGTGTTCCTC CCAATCTCTACGCATTTCACCGCTACACTGGGAATTCC ACTAACCTCTCCGATACTCCAGCTTCCCAGTTTCCTTCC CATCACGGGGTTAAGCCCCGCACTTTTAAGAGGGACT TGGGAAGCCGCCTGCGCGCGCTTTACGCCCAATAATT CCGGACAACGCTTGCCACCTACGTA | 14 |
| # OTU_2494 | *Dialister invisus* | CCTGTTCGCTCCCCACGCTTTCGAGCCTCAGCGTCAGT TACAGACCAGAGAGCCGCTTTCGCCACCGGTGTTCCT CCATATATCTACGCATTTCACCGCTACACATGGAATTC CACTTTCCTCTCCGATACTCCAGCTTCCCAGTTTCCTTC CCATCACGGGGTTAAGCCCCGCACTTTTAAGAGGGAC TTGGGAAGCCGCCTGCGCGCGCTTTACGCCCAATAAT TCCGGACAACGCTTGCCACCTACGTA | 15 |
| # OTU_361 | *D. succinatiphilus* | CCTGTTTGCTCCCCACGCTTTCGCGCCTCAGCGTCAGT TGTCGTCCAGAAAGCCGCCTTCGCCACCGGTGTTCTTC CTAATCTCTACGCATTTCACCGCTACACTAGGAATTCC GCTTTCCTCTCCGATACTCCAGCTCTTCAGTTTCCATCC CATCATGGGGTTAAGCCCCACGCTTTTAAGATGGACT TAAAGGGCCGCCTGCGCGCGCTTTACGCCCAATAATT CCGGACAACGCTTGCCACCTACGTA | 16 |
| # OTU_420 | *D. propionifaciens* | CCTGTTTGCTCCCCACGCTTTCGAGCCTCAGCGTCAGT TATCGTCCAGTAAGCCGCCTTCGCCACTGGTGTTCCTC CTAATATCTACGCATTTCACCGCTACACTAGGAATTCC ACTTTCCTCTCCGATACTCCAGCCTCTCAGTTTCCATCC CATCACGGGGTTAAGCCCCGCACTTTTAAGATGGACT TAAGAAGCCGCCTGCGCGCGCTTTACGCCCAATAATT CCGGACAACGCTTGCCACCTACGTA | 17 |
| # OTU_44 | *D. invisus* | CCTGTTTGCTCCCCACGCTTTCGCGCCTCAGCGTCAGT TTTCGTCCAGAAAGTCGCCTTCGCCACCGGTGTTCTTC CTAATCTCTACGCATTTCACCGCTACACTAGGAATTCC ACTTTCCTCTCCGATACTCCAGCTTCCCAGTTTCCTTCC CATCACGGGGTTAAGCCCCGCACTTTTAAGAGGGACT TGGGAAGCCGCCTGCGCGCGCTTTACGCCCAATAATT CCGGACAACGCTTGCCACCTACGTA | 18 |
| # OTU_497 | *D. invisus* | CCTGTTTGCTCCCCACGCTTTCGAGCCTCAACGTCAGT TACCGTCCAGTAAGCCGCCTTCGCCACTGGTGTTCCTC CTAATATCTACGCATTTCACCGCTACACTAGGAATTCC GCTTACCTCTCCGATACTCCAGCTTCCCAGTTTCCTTCC CATCACGGGGTTAAGCCCCGCACTTTTAAGAGGGACT TGGGAAGCCGCCTGCGCGCGCTTTACGCCCAATAATT CCGGACAACGCTTGCCACCTACGTA | 19 |
| # OTU_515 | *D. propionifaciens* | CCTGTTTGCTACCCACACTTTCGTGCCTCAGCGTCAGT TGTCGTCCAGAAAGTCGCCTTCGCCACCGGTGTTCTTC CTAATCTCTACGCATTTCACCGCTACACTAGGAATTCC ACTTTCCTCTCCGATACTCCAGCCTCCCAGTTTCCATCC CATCACGGGGTTAAGCCCCGCACTTTTAAGATGGACT | 20 |
| | | TAAGAAGCCGCCTGCGCGCGCTTTACGCCCAATAATT CCGGACAACGCTTGCCACCTACGTA | |
| #OTU_685 | *D. succinatiphilus* | CCTGTTTGCTCCCCACGCTTTCGAGCCTCAACGTCAGT CATCGTCCAGCAAGCCGCCTTCGCCACTGGTGTTCCTC CTAATATCTACGCATTTCACCGCTACACTAGGAATTCC ACTTTCCTCTCCGATACTCCAGCTTTCCAGTTTCCATCC CATCACGGGGTTAAGCCCCGCACTTTTAAGATGGACT TAGAAAGCCGCCTGCGCGCGCTTTACGCCCAATAATT CCGGACAACGCTTGCCACCTACGTA | 21 |
| #OTU_882 | *D. invisus* | CCTGTTTGCTCCCCACGCTTTCGAGCCTCAGCGTCAGT TAAAGCCCAGTAAGCCGCCTTCGCCACTGATGTTCCTC CTAATATCTACGCATTTCACCGCTACACTAGGAATTCC ACTTTCCTCTCCGATACTCCAGCTTCCCAGTTTCCTTCC CATCACGGGGTTAAGCCCCGCACTTTTAAGAGGGACT TGGGAAGCCGCCTGCGCGCGCTTTACGCCCAATAATT CCGGACAACGCTTGCCACCTACGTA | 22 |
| # OTU_982 | *D. pseumosintes* | CCCGTTCGCTCCCCTGGCTTTCGCGCCTCAGCGTCAGT TTTCGTCCAGAAAGTCGCCTTCGCCACTGGTGTTCTTC CTAATATCTACGCATTTCACCGCTACACTAGGAATTCC ACTTTCCTCTCCGATACTCCAGCTTCCCAGTTTCCATCC CCTCATGGGGTTGAGCCCCACGCTTTTAAGATGGACT TAAGAAACCGCCTGCGCGCGCTTTACGCCCAATAATTC CGGACAACGCTTGCCACCTACGTA | 23 |
| OTU_984 | *D. pseumosintes* | CCTGTTTGCTCCCCACGCTTTCGCGCCTCAGCGTCAGT TTTCGTCTAGAAAGTCGCCTTCGCCACCGGTGTTCTTC CTAATCTCTACGCATTTCACCGCTACACTAGGAATTCC ACTTTCCCCTCCGATACTCCAGCTTCCCAGTTTCCATCC CCTCATGGGGTTGAGCCCCACGCTTTTAAGATGGACT TAAGAAACCGCCTGCGCGCGCTTTACGCCCAATAATTC CGGACAACGCTTGCCACCTACGTA | 24 |

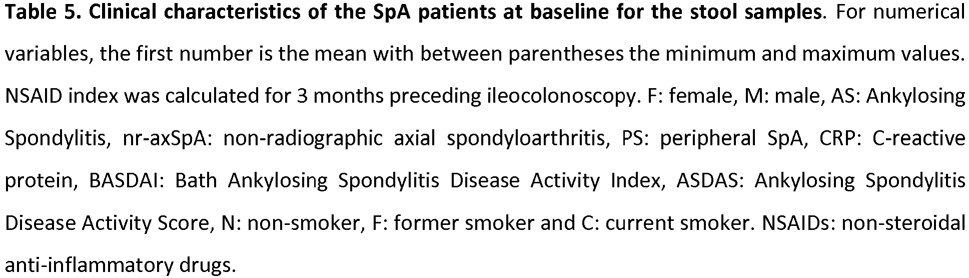

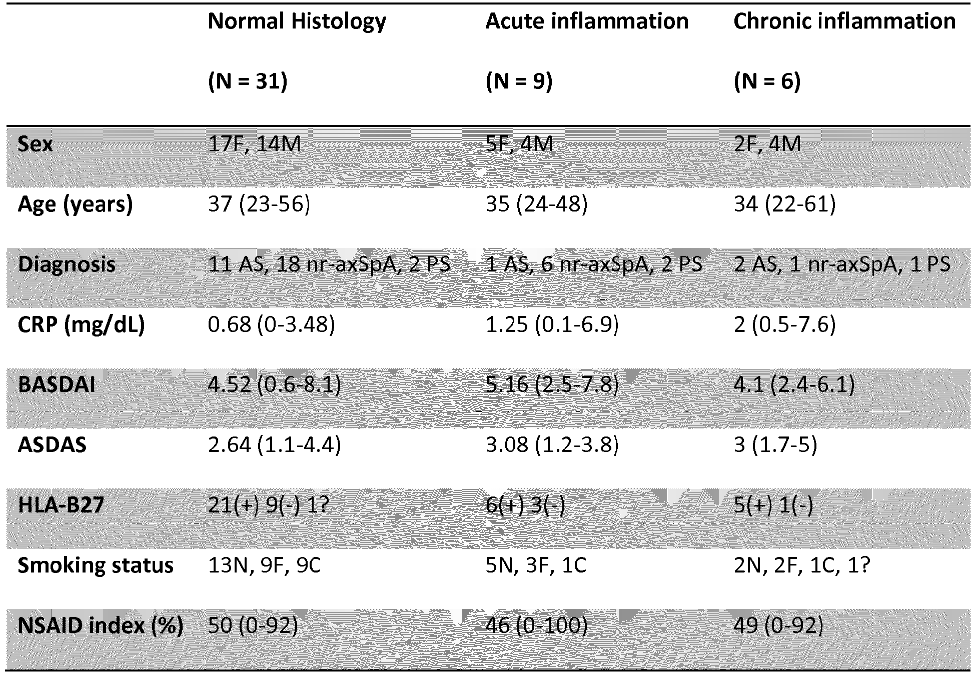

## Claims

1. A method to detect the presence of gut inflammation associated with spondyloarthritis in a patient, comprising the steps of:
- determining the abundance of the *Dialister genus* in a biological sample of said patient, wherein the biological sample is a mucosal biopsy sample, a stool sample, a sample of the lumen content or an oral sample;
- if said abundance in said sample is at least 3-fold higher than that in a control sample of a healthy subject, then concluding that said patient has gut inflammation associated with spondyloarthritis.

2. A method of monitoring the efficacy of a treatment for gut inflammation associated with spondyloarthritis in a patient, said method comprising the steps of:
- determining the abundance of the *Dialister* genus in biological samples collected from said patient on at least two successive time periods, wherein the biological sample is a mucosal biopsy sample, a stool sample, a sample of the lumen content or an oral sample;
- if the abundance of the *Dialister* genus at a later time point of the at least two successive time points is at least 10% lower than the abundance of the *Dialister* genus at an earlier time point of the at least two successive time points, then concluding that said treatment is efficacious;
wherein the earlier time point is at commencement of the treatment and the later time point is during or after cessation of the treatment, or wherein the earlier time point is during the treatment and the later time point is after cessation of the treatment.

3. The method according to any of claims 1-2, wherein the abundance of the *Dialister* genus is determined by amplification products of DNA and/or RNA or by analysis of proteins and/or metabolic products present in the biological sample.

4. The method of any of claims 1-3, wherein said *Dialister* genus is a *Dialister* spp. selected from the list consisting of *Dialister invisus, Dialister propionifaciens, Dialister succinatiphilus, Dialister micraerophilus* and *Dialister pneumosintes.*

5. The method of any of claim 1-4, wherein said *Dialister* genus is a *Dialister* spp. comprising a 16S rRNA sequence comprising SEQ ID No. 17, 18, 20 or 21.

6. The method of any of claims 2-5, wherein said treatment includes a biological therapy, such as TNF-alpha blockers, anti-IL17A monoclonal antibodies.

7. Use of a gut microbiome profile to detect the presence of gut inflammation associated with SpA, wherein said gut microbiome profile comprises an at least 3-fold higher abundance of the Dialister genus compared to an identical gut microbiome profile of a control sample of a healthy subject.

8. The use according to claim 7, wherein the *Dialister* genus is a *Dialister* spp. selected from the list consisting of *Dialister invisus, Dialister propionifaciens, Dialister succinatiphilus, Dialister micraerophilus* and *Dialister pneumosintes.*

9. The use according to any of claim 7-8, wherein said *Dialister* genus is a *Dialister* spp. comprising a 16S rRNA sequence comprising SEQ ID No. 17, 18, 20 or 21.

## Patentansprüche

1. Verfahren zum Nachweisen des Vorliegens einer Darmentzündung, die mit einer Spondyloarthritis assoziiert ist, bei einem Patienten, umfassend die Schritte:
- Bestimmen der Abundanz der Gattung *Dialister* in einer biologischen Probe des Patienten, wobei die biologische Probe eine Schleimhautbiopsieprobe, eine Stuhlprobe, eine Probe des Lumeninhalts oder eine Mundprobe ist;
- wenn die Abundanz in der Probe um mindestens das 3-fache höher als die in einer Kontrollprobe eines gesunden Probanden ist, Folgern, dass der Patient eine Darmentzündung aufweist, die mit einer Spondyloarthritis assoziiert ist.

2. Verfahren zum Überwachen der Wirksamkeit einer Behandlung einer Darmentzündung, die mit einer Spondyloarthritis assoziiert ist, bei einem Patienten, wobei das Verfahren die Schritte umfasst:
- Bestimmen der Abundanz der Gattung *Dialister* in biologischen Proben, die von dem Patienten in mindestens zwei sukzessiven Zeiträumen genommen werden, wobei die biologische Probe eine Schleimhautbiopsieprobe, eine Stuhlprobe, eine Probe des Lumeninhalts oder eine Mundprobe ist;
- wenn die Abundanz der Gattung *Dialister* zu einem späteren Zeitpunkt der mindestens zwei sukzessiven Zeiträume um mindestens 10 % niedriger als die Abundanz der Gattung *Dialister* zu einem früheren Zeitpunkt der mindestens zwei sukzessiven Zeiträume ist, Folgern, dass die Behandlung wirksam ist;
wobei der frühere Zeitpunkt zum Beginn der Behandlung ist und der spätere Zeitpunkt während der oder nach Beendigung der Behandlung ist oder wobei der frühere Zeitpunkt während der Behandlung ist und der spätere Zeitpunkt nach Beendigung der Behandlung ist.

3. Verfahren nach einem der Ansprüche 1-2, wobei die Abundanz der Gattung *Dialister* durch Amplifikationsprodukte von DNA und/oder RNA oder durch Analyse von Proteinen und/oder Stoffwechselprodukten, die in der biologischen Probe vorliegen, bestimmt wird.

4. Verfahren nach einem der Ansprüche 1-3, wobei die Gattung *Dialister* eine *Dialister* spp. ist, die aus der Liste bestehend aus *Dialister invisus, Dialister propionicifaciens, Dialister succinatiphilus, Dialister micraerophilus* und *Dialister pneumosintes* ausgewählt ist.

5. Verfahren nach einem der Ansprüche 1-4, wobei die Gattung *Dialister* eine *Dialister* spp. ist, die eine 16S-rRNA-Sequenz umfasst, die SEQ ID Nr. 17, 18, 20 oder 21 umfasst.

6. Verfahren nach einem der Ansprüche 2-5, wobei die Behandlung eine biologische Therapie beinhaltet, wie TNF-alpha-Blocker, monoklonale Anti-IL17A-Antikörper.

7. Verwendung eines Darmmikrobiomprofils zum Nachweisen des Vorliegens einer Darmentzündung, die mit einer SpA assoziiert ist, wobei das Darmmikrobiomprofil eine um mindestens das 3-fache höhere Abundanz der Gattung *Dialister* im Vergleich zu einem identischen Darmmikrobiomprofil einer Kontrollprobe eines gesunden Probanden umfasst.

8. Verwendung nach Anspruch 7, wobei die Gattung *Dialister* eine *Dialister* spp. ist, die aus der Liste bestehend aus *Dialister invisus, Dialister propionicifaciens, Dialister succinatiphilus, Dialister micraerophilus* und *Dialister pneumosintes* ausgewählt ist.

9. Verwendung nach einem der Ansprüche 7-8, wobei die Gattung *Dialister* eine *Dialister* spp. ist, die eine 16S-rRNA-Sequenz umfasst, die SEQ ID Nr. 17, 18, 20 oder 21 umfasst.

## Revendications

1. Procédé de détection de la présence d'inflammation intestinale associée à une spondylarthrite chez un patient, comprenant les étapes consistant à :
- déterminer l'abondance du genre *Dialister* dans un échantillon biologique dudit patient, dans lequel l'échantillon biologique est un échantillon de biopsie muqueuse, un échantillon de selles, un échantillon du contenu de lumière ou un échantillon oral ;
- si ladite abondance dans ledit échantillon est au moins 3 fois supérieure à celle dans un échantillon témoin d'un sujet sain, conclure alors que ledit patient a de l'inflammation intestinale associée à une spondylarthrite.

2. Procédé de surveillance de l'efficacité d'un traitement pour de l'inflammation intestinale associée à une spondylarthrite chez un patient, ledit procédé comprenant les étapes consistant à :
- déterminer l'abondance du genre *Dialister* dans des échantillons biologiques recueillis auprès dudit patient sur au moins deux périodes de temps successives, dans lequel l'échantillon biologique est un échantillon de biopsie muqueuse, un échantillon de selles, un échantillon du contenu de lumière ou un échantillon oral ;
- si l'abondance du genre *Dialister* à un moment ultérieur des au moins deux moments successifs est d'au moins 10 % inférieure à l'abondance du genre *Dialister* à un moment antérieur des au moins deux moments successifs, conclure alors que ledit traitement est efficace ;
dans lequel le moment antérieur est au début du traitement et le moment ultérieur est au cours ou après arrêt du traitement, ou dans lequel le moment antérieur est au cours du traitement et le moment ultérieur est après arrêt du traitement.

3. Procédé selon l'une quelconque des revendications 1 et 2, dans lequel l'abondance du genre *Dialister* est déterminée par des produits d'amplification d'ADN et/ou d'ARN ou par analyse de protéines et/ou produits métaboliques présents dans l'échantillon biologique.

4. Procédé selon l'une quelconque des revendications 1 à 3, dans lequel ledit genre *Dialister* est un *Dialister* spp. sélectionné parmi la liste constituée de *Dialister invisus, Dialister propionifaciens, Dialister succinatiphilus, Dialister micraerophilus* et *Dialister pneumosintes.*

5. Procédé selon l'une quelconque des revendications 1 à 4, dans lequel ledit genre *Dialister* est un *Dialister* spp. comprenant une séquence d'ARNr 16S comprenant SEQ ID N° 17, 18, 20 ou 21.

6. Procédé selon l'une quelconque des revendications 2 à 5, dans lequel ledit traitement inclut une thérapie biologique, telle que des bloqueurs du TNF-alpha, des anticorps monoclonaux anti-IL17A.

7. Utilisation d'un profil de microbiome intestinal pour détecter la présence d'inflammation intestinale associée à une SpA, dans laquelle ledit profil de microbiome intestinal comprend une abondance au moins 3 fois supérieure du genre Dialister par rapport à un profil de microbiome intestinal identique d'un échantillon témoin d'un sujet sain.

8. Utilisation selon la revendication 7, dans laquelle le genre *Dialister* est un *Dialister* spp. sélectionné parmi la liste constituée de *Dialister invisus, Dialister propionifaciens, Dialister succinatiphilus, Dialister micraerophilus* et *Dialister pneumosintes.*

9. Utilisation selon l'une quelconque des revendications 7 et 8, dans laquelle ledit genre *Dialister* est un *Dialister* spp. comprenant une séquence d'ARNr 16S comprenant SEQ ID N° 17, 18, 20 ou 21.
